(19) Europäisches Patentamt
European Patent Office
Office européen des brevets

(11) **EP 2 979 635 B1**

(12) **EUROPEAN PATENT SPECIFICATION**

(45) Date of publication and mention
of the grant of the patent:
**24.10.2018 Bulletin 2018/43**

(51) Int Cl.:
*A61B 5/16* *(2006.01)*          *G06F 3/01* *(2006.01)*
*G06Q 30/02* *(2012.01)*          *A61B 3/113* *(2006.01)*

(21) Application number: **15177666.3**

(22) Date of filing: **21.07.2015**

(54) **DIAGNOSIS SUPPORTING DEVICE, DIAGNOSIS SUPPORTING METHOD, AND COMPUTER-READABLE RECORDING MEDIUM**

DIAGNOSEUNTERSTÜTZUNGSVORRICHTUNG, DIAGNOSEUNTERSTÜTZUNGSVERFAHREN UND COMPUTERLESBARES AUFZEICHNUNGSMEDIUM

DISPOSITIF ET PROCÉDÉ DE SUPPORT DE DIAGNOSTIC ET SUPPORT D'ENREGISTREMENT LISIBLE PAR ORDINATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.07.2014   JP 2014156812**
**31.07.2014   JP 2014156872**
**28.11.2014   JP 2014242033**

(43) Date of publication of application:
**03.02.2016   Bulletin 2016/05**

(73) Proprietor: **JVC KENWOOD Corporation**
**Yokohama-shi**
**Kanagawa 221-0022 (JP)**

(72) Inventor: **SHUDO, Katsuyuki**
**Yokohama-shi,**
**Kanagawa, 221-0022 (JP)**

(74) Representative: **Wimmer, Hubert**
**WAGNER & GEYER**
**Gewürzmühlstrasse 5**
**80538 München (DE)**

(56) References cited:
**EP-A1- 2 754 397          JP-A- 2014 068 933**
**US-A1- 2010 004 977          US-A1- 2014 142 397**

**Description**

BACKGROUND OF THE INVENTION

1. Field of the Invention

The present invention relates to a diagnosis supporting device, a diagnosis supporting method and a computer-readable recording medium.

2. Description of the Related Art

It is often the case that developmentally disabled people have characteristics of deviated gazing points or difficulties in understanding causal relations. It's important in providing care and education (training) on developmentally disabled people to understand what point they gaze at to obtain information in order to understand a causal relation, whether they cannot understand the causal relation despite gazing at, or the like. Conventional techniques are described in Japanese Patent Application Laid-open No. 2011-206542 and Pierce K et al., "Preference for Geometric Patterns Early in Life as a Risk Factor for Autism," Arch Gen Psychiatry. 2011 Jan; 68 (1): 101-109, for example.

Also US 2010/0004977 A discloses a method and system for measuring the biometric (physically, behaviorally, biologically and self-report based) responses of an audience to a presentation or interactive experience that provides a sensory stimulating experience and determining a measure of the level and pattern of engagement of that audience and impact of the presentation or interactive experience by eye tracking to determine areas of the presentation that correspond to high and low levels of biometric responses suggesting high and low levels of visual impact.

Furthermore, EP 2754397 A discloses a predetermined analysis that means calculation, from the information of eye-gaze positions and information of image to be displayed, as to what position (area) of the areal information in the information of image to be displayed the subject looks at and in what frequency the eye-gaze movement between some specific areas occurs, and presentation of the results of the calculation by counting the number of eye-gaze positions found in each of illusionary area S and non-illusionary area N.

However, the conventional methods cannot give an understanding of what point developmentally disabled people gaze at to obtain information in order to understand a causal relation, whether they cannot understand the causal relation despite gazing at, or the like. For this reason, the conventional methods may fail to appropriately support diagnosis, and a diagnosis supporting method with higher precision has been demanded.

SUMMARY OF THE INVENTION

It is an object of the present invention to at least partially solve the problems in the conventional technology.

In accordance with the present invention, a diagnosis supporting device, a diagnosis supporting method, and a non-transitory computer-readable recording medium as set forth in claims 1, 6 and 7 is provided. Further embodiments of the invention are inter alia claimed in the dependent claims. In particular, there is provided a diagnosis supporting device that includes a display, an imaging unit configured to image a subject, a visual line detector configured to detect a visual line direction of the subject from an image imaged by the imaging unit, a visual point detector configured to detect a visual point of the subject in a display area of the display based on the visual line direction, an output controller configured to display a diagnostic image comprising a first image representing a cause of a certain event and a second image representing a result of the cause, and an explanation screen representing a causal relation between the first image and the second image on the display in the order of the first image, the second image, and the explanation screen on the display, and an evaluator configured to calculate an evaluation value of the subject based on the visual point detected by the visual point detector when the diagnostic image is displayed.

The above and other objects, features, advantages and technical and industrial significance of this invention will be better understood by reading the following detailed description of presently preferred embodiments of the invention, when considered in connection with the accompanying drawings.

BRIEF DESCRIPTION OF THE DRAWINGS

FIG. 1 is a diagram illustrating an example of an arrangement of a display, a stereo camera, an infrared light source of an embodiment;
FIG. 2 is a diagram illustrating an example of the arrangement of the display, the stereo camera, the infrared light source, and a subject of the present embodiment;

FIG. 3 is a diagram illustrating an outline of functions of a diagnosis supporting device;

FIG. 4 is a block diagram illustrating an example of detailed functions of the units illustrated in FIG. 3;

FIG. 5 is a diagram illustrating an outline of processing performed by a diagnosis supporting device of the present embodiment;

FIG. 6 is an illustrative diagram illustrating a difference between a method of using two light sources and the present embodiment of using one light source;

FIG. 7 is a diagram for illustrating calculation processing for calculating the distance between a pupil center position and a corneal curvature center position;

FIG. 8 is a flowchart illustrating an example of calculation processing of the present embodiment;

FIG. 9 is a diagram illustrating a method for calculating the position of a corneal curvature center using a distance determined in advance;

FIG. 10 is a flowchart illustrating an example of visual line detection processing of the present embodiment;

FIG. 11 is a diagram for illustrating calculation processing of Modification 1;

FIG. 12 is a flowchart illustrating an example of the calculation processing of Modification 1;

FIG. 13 is a diagram illustrating an example of a diagnostic image used in the present embodiment;

FIG. 14 is a diagram illustrating an example of the diagnostic image used in the present embodiment;

FIG. 15 is a diagram illustrating an example of the diagnostic image used in the present embodiment;

FIG. 16 is a diagram illustrating an example of the diagnostic image used in the present embodiment;

FIG. 17 is a flowchart illustrating an example of diagnosis support processing of the present embodiment;

FIG. 18 is a diagram illustrating an example of a selection screen for selecting a primary answer;

FIG. 19 is a diagram illustrating an example of a right answer screen for displaying a right answer;

FIG. 20 is a diagram illustrating an example of an explanation screen;

FIG. 21 is a flowchart illustrating an example of gazing point detection processing;

FIG. 22 is a flowchart illustrating an example of analysis processing;

FIG. 23 is a diagram illustrating examples of a causal relation;

FIG. 24 is a flowchart illustrating an example of verification processing that verifies and displays effects of care and education;

FIG. 25 is a diagram for illustrating an example of a method for determining changes in measurement data;

FIG. 26 is a flowchart illustrating an example of analysis processing when a moving image is displayed;

FIG. 27 is a flowchart illustrating an example of training support processing of Modification 2;

FIG. 28 is a diagram illustrating an example of a menu screen of Modification 2;

FIG. 29 is a diagram illustrating an example of a still image 1 displayed in Modification 2;

FIG. 30 is a diagram illustrating an example of a still image 2 displayed in Modification 2;

FIG. 31 is a diagram illustrating an example of a selection screen of Modification 2;

FIG. 32 is a diagram illustrating an example of a right answer screen of Modification 2;

FIG. 33 is a diagram illustrating an example of an explanation screen of Modification 2;

FIG. 34 is a diagram illustrating an example of a reproduction screen of Modification 2; and

FIG. 35 is a diagram illustrating an example of implementing a training supporting device by a notebook PC.

## DETAILED DESCRIPTION OF THE PREFERRED EMBODIMENTS

The following describes embodiments of a diagnosis supporting device, a diagnosis supporting method, a computer-readable recording medium for supporting diagnosis according to the present invention in detail with reference to the drawings. The present invention is not limited by the embodiments. Although the following describes a case in which the diagnosis supporting device is used as the diagnosis supporting device that supports diagnosis of a developmental disorder or the like using a visual line detection result and can also be used for training, applicable devices are not limited to this.

The diagnosis supporting device of the present embodiment displays an image (video) indicating before and after an event, measures a dwell time at a position of a gazing point, and performs evaluation computation. A continuous moving image containing a scene representing a cause and a scene indicating before and after the event is displayed as an explanation image indicating a causal relation between the cause and the event. With this configuration, efficient training support that is easily understood by trainees is achieved.

The diagnosis supporting device of the present embodiment detects a visual line using an illuminator placed at one position. The diagnosis supporting device is not limited to the above embodiment. The diagnosis supporting device of the present embodiment, using a result measured by causing a subject to gaze at one point before visual line detection, calculates a corneal curvature center position with high accuracy.

The illuminator includes a light source and is a component that can apply light to an eyeball of the subject. The light source is, for example, an element that emits light such as a light emitting diode (LED). The light source may include one LED or include a plurality of LEDs combined and arranged at one position. The following may use a "light source" as a term thus representing the illuminator.

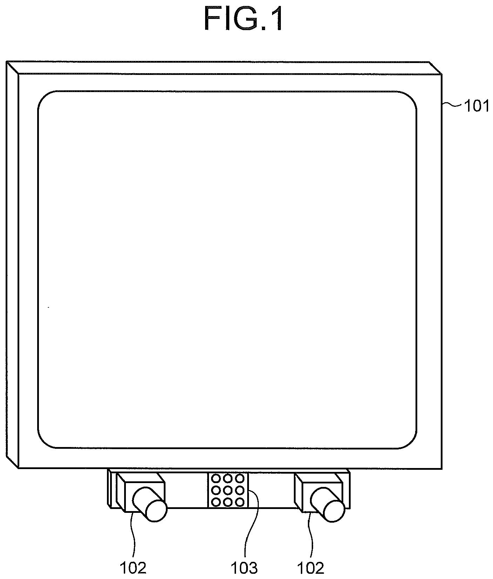

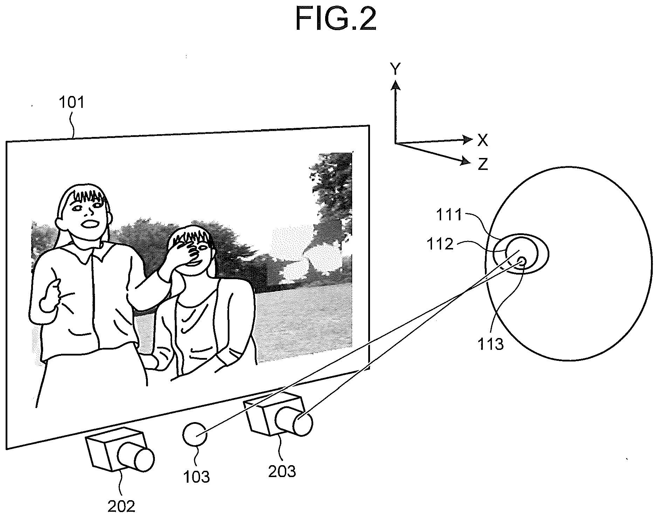

FIGS. 1 and 2 are diagrams illustrating an example of an arrangement of a display, a stereo camera, an infrared light source, and a subject of the present embodiment.

As illustrated in FIG. 1, this diagnosis supporting device 100 of the present embodiment includes a display 101, a stereo camera 102 corresponding to an imaging unit, and an LED light source 103. The stereo camera 102 is arranged below the display 101. The LED light source 103 is arranged at the central position between two cameras included in the stereo camera 102. The LED light source 103 is a light source that emits near-infrared rays with a wavelength of, for example, 850 nm. FIG. 1 illustrates an example of constituting the LED light source 103 (the illuminator) by nine LEDs. The stereo camera 102 uses lenses that can transmit near-infrared rays with a wavelength of 850 nm therethrough.

As illustrated in FIG. 2, the stereo camera 102 includes a right camera 202 and a left camera 203. The LED light source 103 applies near-infrared rays toward an eyeball 111 of the subject. In an image captured by the stereo camera 102, a pupil 112 reflects with low brightness and appears dark, whereas a corneal reflex 113 occurring as a virtual image within the eyeball 111 reflects with high brightness and appears bright. Positions of the pupil 112 and the corneal reflex 113 on the image can therefore be acquired by the two cameras (the right camera 202 and the left camera 203) separately.

From the positions of the pupil 112 and the corneal reflex 113 obtained by the two cameras, three-dimensional world coordinate values of the positions of the pupil 112 and the corneal reflex 113 are calculated. In the present embodiment, as three-dimensional world coordinates, with the central position of a screen of the display 101 as the point of origin, the up-and-down direction is a Y coordinate (the upper side is +), the lateral direction is an X coordinate (the right side viewed from the front is +), and the depth direction is a Z coordinate (the near side is +).

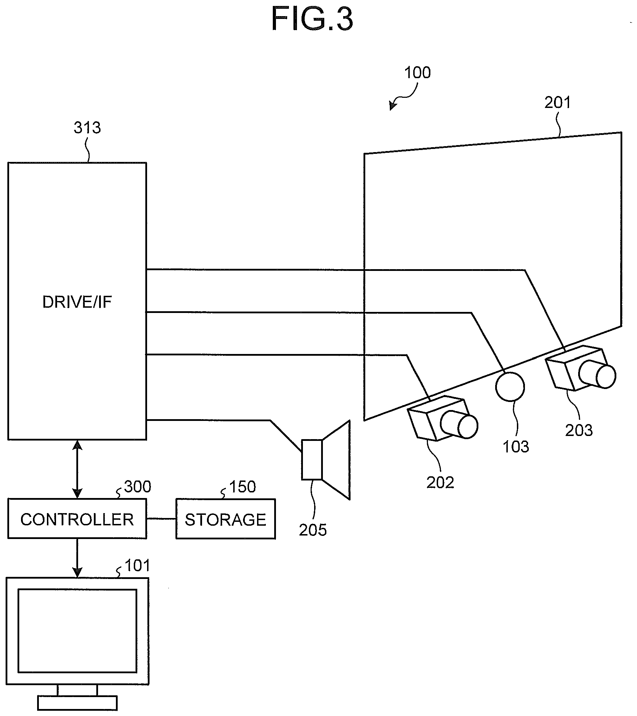

FIG. 3 is a diagram illustrating an outline of functions of the diagnosis supporting device 100. FIG. 3 illustrates part of the configuration illustrated in FIG. 1 and FIG. 2 and a configuration used for the drive or the like of the configuration. As illustrated in FIG. 3, the diagnosis supporting device 100 includes the right camera 202, the left camera 203, the LED light source 103, a speaker 205, a drive-and-interface (I/F) 313, a controller 300, a storage 150, and the display 101. Although FIG. 3 illustrates a display screen 201 in such a manner that position relation with the right camera 202 and the left camera 203 is easy to understand, the display screen 201 is a screen displayed on the display 101. The driver and the IF may be integral or separate.

The speaker 205 functions as a voice output unit that outputs a voice for calling subject's attention or the like during calibration or the like.

The drive/IF 313 drives units included in the stereo camera 102. The drive/IF 313 serves as an interface between the units included in the stereo camera 102 and the controller 300.

The controller 300 is implemented by a computer or the like including, for example, a controller such as a central processing unit (CPU), storage devices such as a read only memory (ROM) and a random access memory (RAM), a communication I/F that connects to a network to perform communication, and a bus that connects the units to each other.

The storage 150 stores therein various types of information such as control programs, measurement results, and diagnosis support results. The storage 150, for example, stores therein images or the like to be displayed on the display 101. The display 101 displays various types of information such as images to be diagnosed.

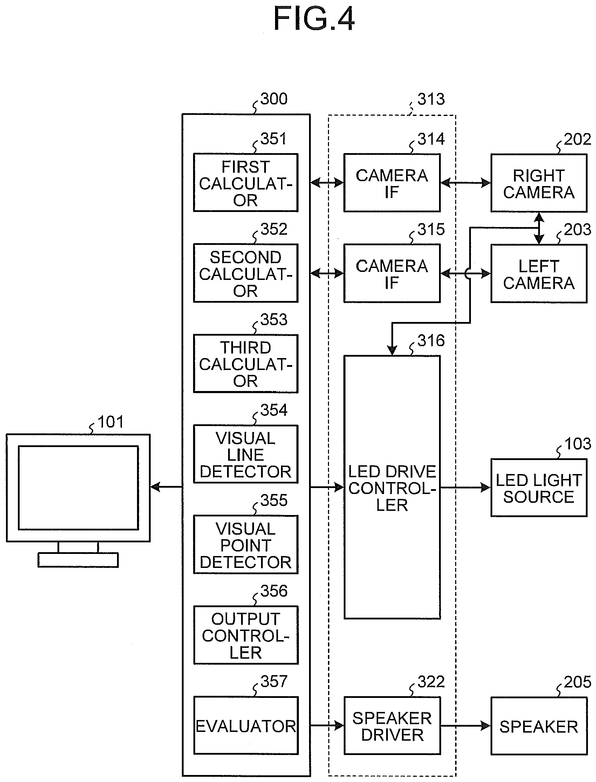

FIG. 4 is a block diagram illustrating an example of detailed functions of the units illustrated in FIG. 3. As illustrated in FIG. 4, the display 101 and the drive/IF 313 are connected to the controller 300. The drive/IF 313 includes camera IFs 314 and 315, an LED drive controller 316, and a speaker driver 322.

The right camera 202 and the left camera 203 are connected to the drive/IF 313 via the camera IFs 314 and 315, respectively. The drive/IF 313 drives the cameras, thereby imaging the subject.

The speaker driver 322 drives the speaker 205. The diagnosis supporting device 100 may include an interface (a printer IF) for connecting to a printer as a printing unit. The printer may be incorporated into the diagnosis supporting device 100.

The controller 300 controls the entire diagnosis supporting device 100. The controller 300 includes a first calculator 351, a second calculator 352, a third calculator 353, a visual line detector 354, a visual point detector 355, an output controller 356, and an evaluator 357. A visual line detection supporting device that detects a visual line only needs to include at least the first calculator 351, the second calculator 352, the third calculator 353, and the visual line detector 354.

Each of the components (the first calculator 351, the second calculator 352, the third calculator 353, the visual line detector 354, the visual point detector 355, the output controller 356, and the evaluator 357) included in the controller 300 may be implemented by software (a computer program), may be implemented by a hardware circuit, or may be implemented by using both the software and the hardware circuit.

When each of the components is implemented by the program, the program is recorded in a computer-readable recording medium such as a compact disc read only memory (CD-ROM), a flexible disk (FD), a compact disc recordable (CD-R), and a digital versatile disc (DVD) as an installable or executable file and is provided as a computer program product. The program may be stored in a computer connected to a network such as the Internet and provided by being downloaded via the network. The program may be provided or distributed via a network such as the Internet. The program may be embedded and provided in a ROM, for example.

The first calculator 351 calculates a position (a first position) of a pupil center indicating the center of a pupil from an image of an eyeball imaged by the stereo camera 102. The second calculator 352 calculates a position (a second position) of a corneal reflex center indicating the center of a corneal reflex from the taken image of the eyeball. The first calculator 351 and the second calculator 352 correspond to a position detector that detects the first position indicating the center of the pupil and the second position indicating the center of the corneal reflex.

The third calculator 353 calculates a corneal curvature center (a fourth position) from a line (a first line) connecting between the LED light source 103 and the corneal reflex center. The third calculator 353, for example, calculates a position that is on the line and the distance of which from the corneal reflex center is a certain value as the corneal curvature center. The certain value can be a value determined in advance from a general corneal curvature radius value or the like.

The corneal curvature radius value can have individual differences, and when the corneal curvature center is calculated using the value determined in advance, a large error may possibly occur. Given this situation, the third calculator 353 may calculate the corneal curvature center in consideration of the individual differences. In this case, the third calculator 353 first, using the pupil center and the corneal reflex center calculated when the subject is made to gaze at a target position (a third position), calculates a point of intersection between a line (a second line) connecting between the pupil center and the target position and a line (the first line) connecting between the corneal reflex center and the LED light source 103. The third calculator 353 then calculates a distance (a first distance) between the pupil center and the calculated point of intersection and stores the distance, for example, in the storage 150.

The target position may be a position that is determined in advance and the three-dimensional world coordinate values of which can be calculated. The central position (the point of origin of the three-dimensional world coordinates) of the display screen 201, for example, can be the target position. In this case, the output controller 356, for example, displays an image (a target image) at which the subject is made to gaze at the target position (center) of the display screen 201. With this configuration, the subject can be made to gaze at the target position.

The target image may be any image so long as it is an image at which the subject can be made to gaze. Examples of the target image include an image the display manner such as brightness and color of which changes and an image the display manner of which is different from the other areas.

The target position is not limited to the center of the display screen 201 and may be any position. The center of the display screen 201 as the target position minimizes the distance to any end of the display screen 201. This arrangement can reduce a measurement error at the time of visual line detection, for example.

The processing up to the calculation of the distance is performed in advance, for example, before starting actual visual line detection. At the time of the actual visual line detection, the third calculator 353 calculates a position that is on the line connecting between the LED light source 103 and the corneal reflex center and the distance from the pupil center of which is the distance calculated in advance as the corneal curvature center. The third calculator 353 corresponds to a calculator that calculates a corneal curvature center (the fourth position) from the position of the LED light source 103, a certain position (the third position) indicating the target image on the display 101, the position of the pupil center, and the position of the corneal reflex center.

The visual line detector 354 detects a visual line of the subject from the pupil center and the corneal curvature center. The visual line detector 354, for example, detects a direction from the corneal curvature center toward the pupil center as a visual line direction of the subject.

The visual point detector 355 detects a visual point of the subject using the detected visual line direction. The visual point detector 355, for example, detects a visual point (a gazing point), which is a point at which the subject gazes on the display screen 201. The visual point detector 355 detects a point of intersection between a visual line vector represented in a three-dimensional world coordinate system as in, for example, FIG. 2 and an XY-plane as the gazing point of the subject.

The output controller 356 controls output of various types of information for the display 101 and the speaker 205. The output controller 356, for example, outputs the target image at the target position on the display 101. The output controller 356 controls output of a diagnostic image, evaluation results by the evaluator 357, or the like to the display 101.

The diagnostic image may be an image appropriate for evaluation processing based on a visual line (visual point) detection result. When a developmental disorder is diagnosed, for example, a diagnostic image containing an image (a geometrical pattern video or the like) that subjects of the developmental disorder like and other images (portrait videos or the like) may be used.

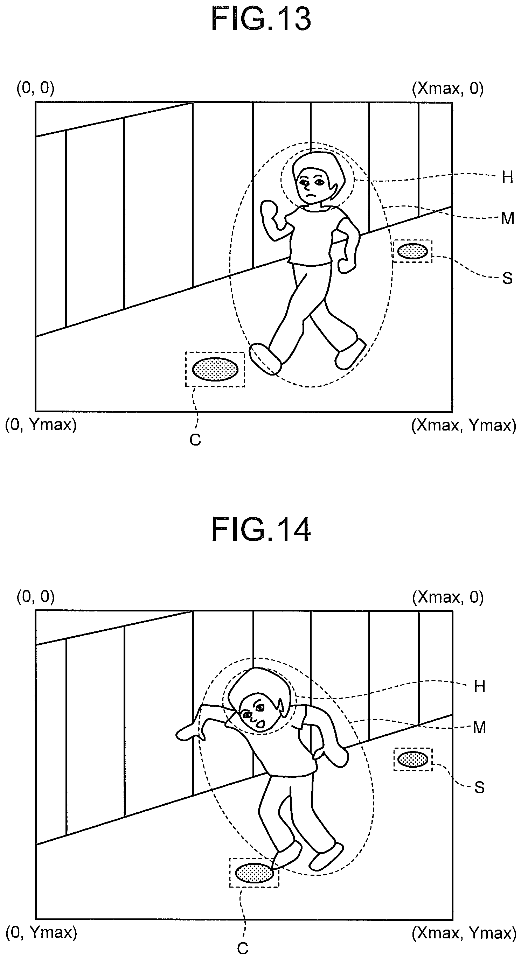

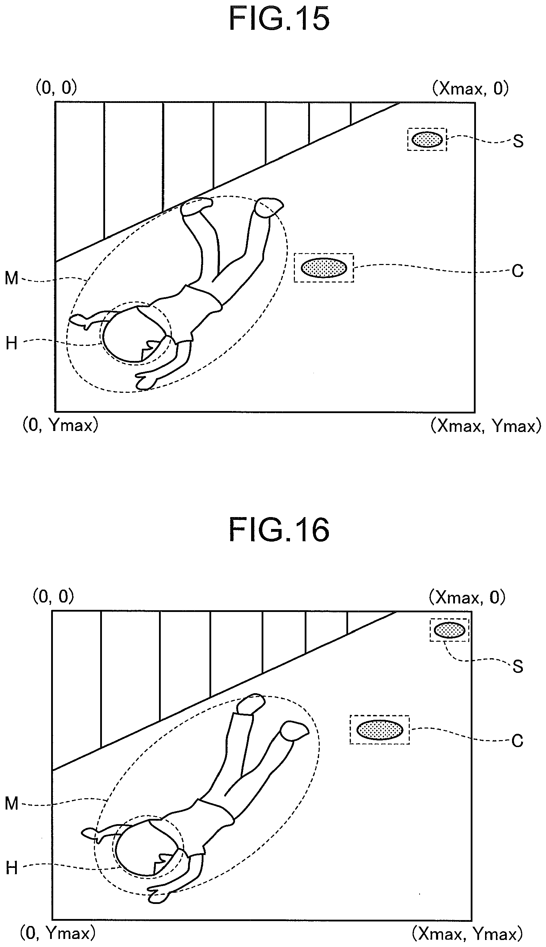

The evaluator 357 performs evaluation processing based on the diagnostic image and the gazing point detected by the visual point detector 355. When a developmental disorder is diagnosed, for example, the evaluator 357 analyzes the diagnostic image and the gazing point and evaluates whether the image that subjects of the developmental disorder like has been gazed at. The evaluator 357, for example, calculates an evaluation value based on the position of the gazing point by the subject when such diagnostic images as illustrated in FIG. 13 and FIG. 16 described below are displayed. A specific example of a method for calculating the evaluation value will be described below. The evaluator 357 only needs to calculate the evaluation value based on the diagnostic image and the gazing point, and the method for calculating the evaluation value is not limited to the present embodiment.

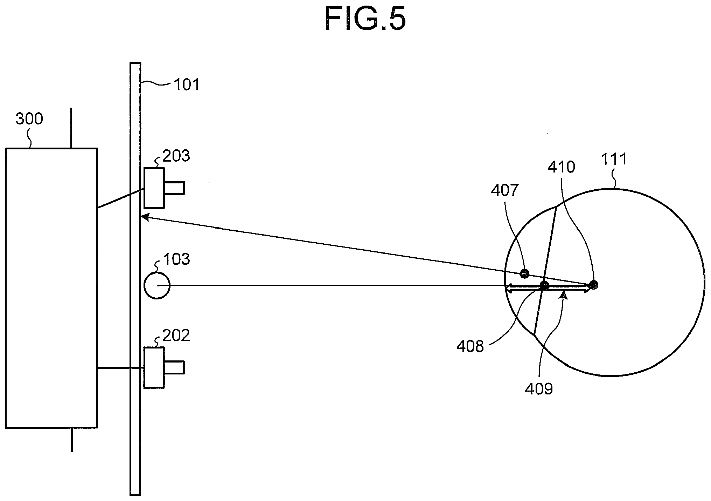

FIG. 5 is a diagram illustrating an outline of processing performed by the diagnosis supporting device 100 of the present embodiment. The components described in FIG. 1 to FIG. 4 are attached with the same signs, and descriptions thereof are omitted.

A pupil center 407 and a corneal reflex center 408 represent the center of a pupil and the center of a corneal reflex point, respectively, detected when the LED light source 103 is turned on. A corneal curvature radius 409 represents the distance from a corneal surface to a corneal curvature center 410.

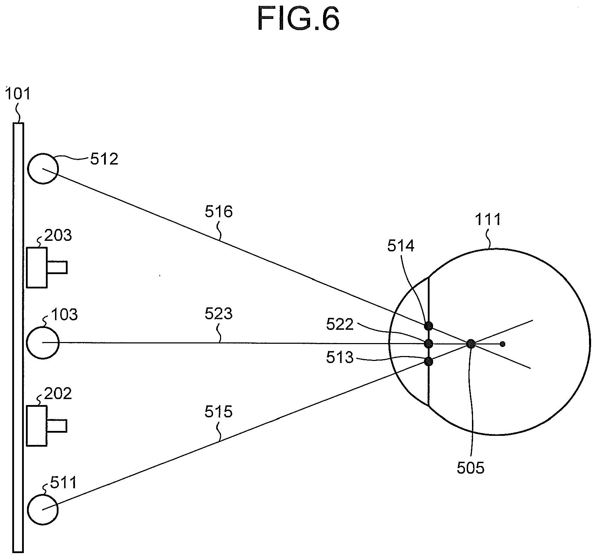

FIG. 6 is an illustrative diagram illustrating a difference between a method (hereinafter, referred to as a method A) using two light sources (the illuminator) and the present embodiment using one light source (the illuminator). The components described in FIG. 1 to FIG. 4 are attached with the same signs, and descriptions thereof are omitted.

The method A uses two LED light sources 511 and 512 in place of the LED light source 103. The method A calculates a point of intersection between a line 515 connecting between a corneal reflex center 513 illuminated by the LED light source 511 and the LED light source 511 and a line 516 connecting between a corneal reflex center 514 illuminated by the LED light source 512 and the LED light source 512. This point of intersection is a corneal curvature center 505.

In contrast, considered in the present embodiment is a line 523 connecting between a corneal reflex center 522 illuminated by the LED light source 103 and the LED light source 103. The line 523 passes through the corneal curvature center 505. It is known that the curvature radius of the cornea has nearly a constant value with less influence of individual differences. From this fact, the corneal curvature center illuminated by the LED light source 103 is present on the line 523 and can be calculated using the general curvature radius value.

However, when a visual point is calculated using the position of the corneal curvature center determined using the general curvature radius value, a visual point position deviates from the original position due to individual differences in the eyeball, which may fail to perform accurate visual point detection.

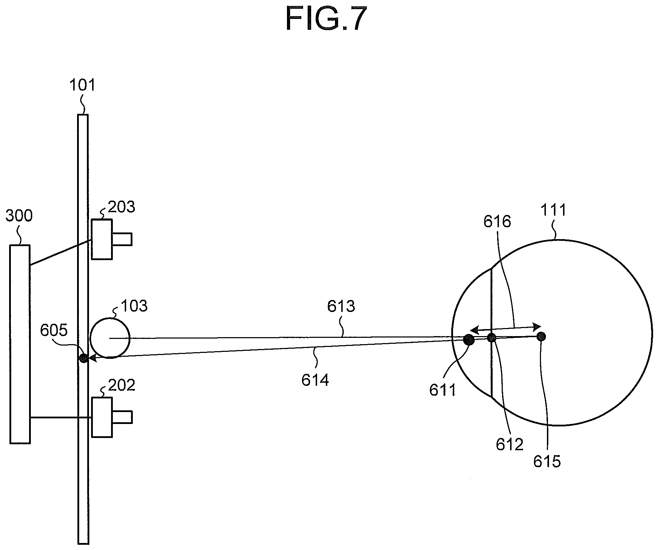

FIG. 7 is a diagram for illustrating calculation processing for calculating the corneal curvature center position and the distance between a pupil center position and a corneal curvature center position before performing visual point (visual line) detection. The components described in FIG. 1 to FIG. 4 are attached with the same signs, and descriptions thereof are omitted.

A target position 605 is a position at which the target image or the like is displayed at one point on the display 101 and at which the subject is made to gaze. In the present embodiment, the target position 605 is the central position of the screen of the display 101. A line 613 is a line connecting between the LED light source 103 and a corneal reflex center 612. A line 614 is a line connecting between the target position 605 (the gazing point) at which the subject gazes and a pupil center 611. A corneal curvature center 615 is a point of intersection between the line 613 and the line 614. The third calculator 353 calculates and stores therein a distance 616 between the pupil center 611 and the corneal curvature center 615.

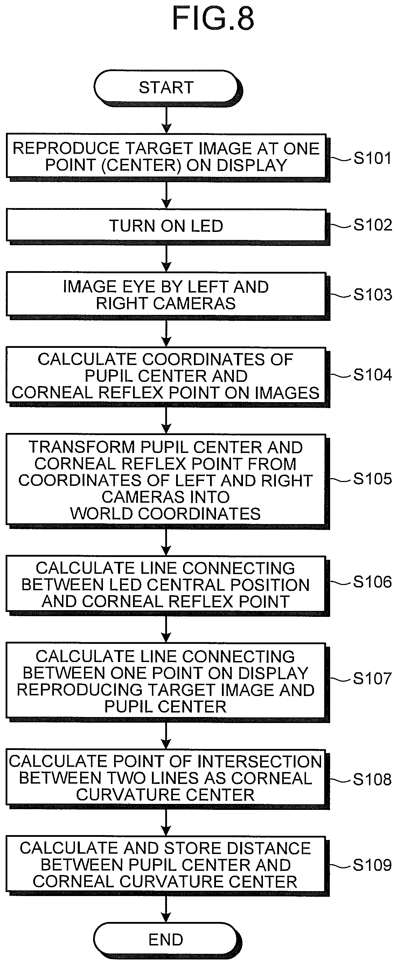

FIG. 8 is a flowchart illustrating an example of calculation processing of the present embodiment.

First, the output controller 356 reproduces the target image at one point on the screen of the display 101 (Step S101) and causes the subject to gaze at the one point. Next, the controller 300 turns on the LED light source 103 toward an eye of the subject using the LED drive controller 316 (Step S102). The controller 300 images the eye of the subject by the left and right cameras (the right camera 202 and the left camera 203) (Step S103).

By the emission from the LED light source 103, a pupil part is detected as a dark part (a dark pupil). A virtual image of a corneal reflex occurs as a reflection of the LED emission, and the corneal reflex point (corneal reflex center) is detected as a bright part. Specifically, the first calculator 351 detects the pupil part from the taken image and calculates coordinates indicating the position of the pupil center. The first calculator 351, for example, detects an area of certain brightness or less containing the darkest part in a certain area containing the eye as the pupil part and detects an area of certain brightness or more containing the brightest part as the corneal reflection. The second calculator 352 detects a corneal reflex part from the taken image and calculates coordinates indicating the position of the corneal reflex center. The first calculator 351 and the second calculator 352 calculate the coordinate values for the respective two images acquired by the left and right cameras (Step S104).

The right and left cameras are subjected to camera calibration by a method of stereo calibration in advance in order to acquire three-dimensional world coordinates, and transformation parameters are calculated. The method of stereo calibration may be any of conventionally used methods such as the method using the camera calibration theory by Tsai.

The first calculator 351 and the second calculator 352, using the transformation parameters, transforms the coordinates of the left and right camera into three-dimensional world coordinates of the pupil center and the corneal reflex center (Step S105). The third calculator 353 obtains a line connecting between the determined world coordinates of the corneal reflex center and the world coordinates of a center position of the LED light source 103 (Step S106). Next, the third calculator 353 calculates a line connecting between the world coordinates of the center of the target image displayed at one point on the screen of the display 101 and the world coordinates of the pupil center (Step S107). The third calculator 353 obtains a point of intersection between the line calculated at Step S106 and the line calculated at Step S107 and determines the point of intersection to be the corneal curvature center (Step S108). The third calculator 353 calculates the distance between the pupil center and the corneal curvature center in this situation and stores the distance in the storage 150 or the like (Step S109). The stored distance is used for calculating the corneal curvature center at the time of subsequent visual point (visual line) detection.

The distance between the pupil center and the corneal curvature center when the subject gazes at the one point on the display 101 in the calculation processing is maintained constant within the range of detecting the visual point within the display 101. The distance between the pupil center and the corneal curvature center may be determined from the average of total values calculated during the reproduction of the target image or determined from the average of several values among the values calculated during the reproduction.

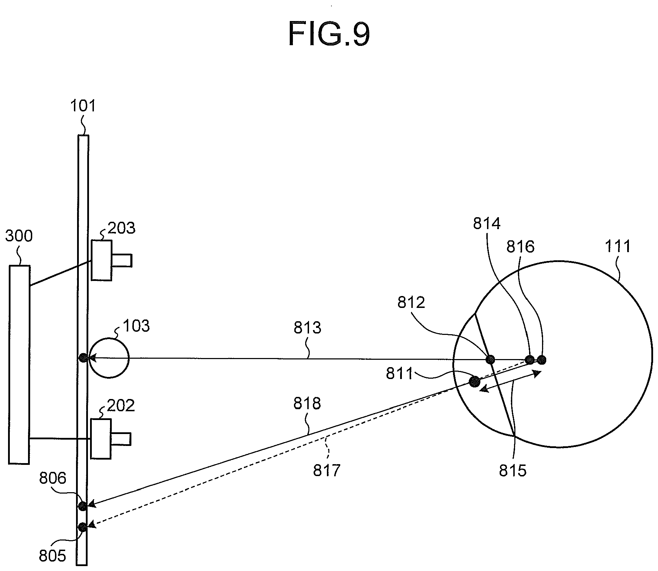

FIG. 9 is a diagram illustrating a method for calculating a corrected position of the corneal curvature center using the distance between the pupil center and the corneal curvature center determined in advance at the time of visual point detection. A gazing point 805 represents a gazing point determined from the corneal curvature center calculated using the general curvature radius value. A gazing point 806 represents a gazing point determined from the corneal curvature center calculated using the distance determined in advance.

A pupil center 811 and a corneal reflex center 812 indicate the position of the pupil center and the position of the corneal curvature center, respectively, calculated at the time of visual point detection. A line 813 is a line connecting between the LED light source 103 and the corneal reflex center 812. A corneal curvature center 814 is the position of the corneal curvature center calculated from the general curvature radius value. A distance 815 is the distance between the pupil center and the corneal curvature center calculated by the advance calculation processing. A corneal curvature center 816 is the position of the corneal curvature center calculated using the distance determined in advance. The corneal curvature center 816 is determined by the corneal curvature center that is present on the line 813 and the distance between the pupil center and the corneal curvature center that is the distance 815. With this determination, a visual line 817 calculated when the general curvature radius value is used is corrected to a visual line 818. The gazing point on the screen of the display 101 is corrected from the gazing point 805 to the gazing point 806.

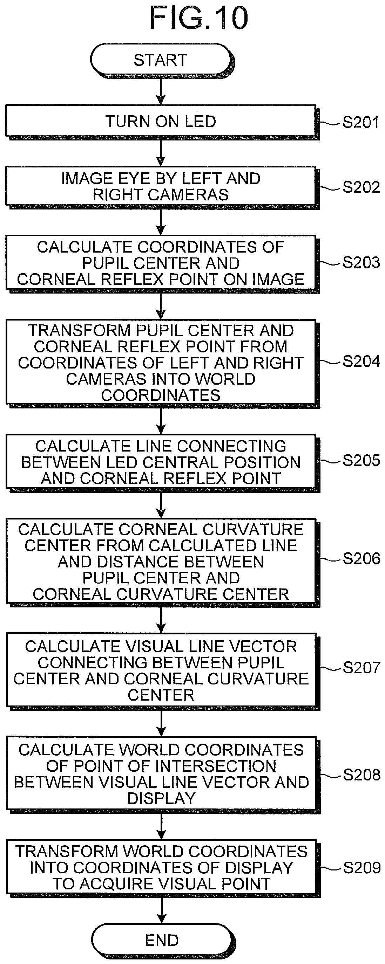

FIG. 10 is a flowchart illustrating an example of visual line detection processing of the present embodiment. The visual line detection processing of FIG. 10 can be performed as processing to detect a visual line in diagnosis processing using the diagnostic image. In the diagnosis processing, in addition to the steps of FIG. 10, processing to display the diagnostic image, evaluation processing by the evaluator 357 using the detection result of the gazing point, and the like are performed.

Steps S201 to S205 are similar to Steps S102 to S106 of FIG. 8, and descriptions thereof are omitted.

The third calculator 353 calculates a position which is on the line calculated at Step S205 and that the distance from the pupil center is equal to the distance determined by the calculation processing in advance, as the corneal curvature center (Step S206).

The visual line detector 354 determines a vector (visual line vector) connecting between the pupil center and the corneal curvature center (Step S207). The vector indicates the visual line direction that the subject is seeing. The visual point detector 355 calculates three-dimensional world coordinate values of a point of intersection between the visual line direction and the screen of the display 101 (Step S208). The values are coordinate values representing the one point on the display 101 at which the subject gazes with the world coordinates. The visual point detector 355 transforms the determined three-dimensional world coordinate values into coordinate values (x, y) represented by a two-dimensional coordinate system of the display 101 (Step S209). With this transformation, the visual point (gazing point) on the display 101 at which the subject gazes can be calculated.

Modification 1

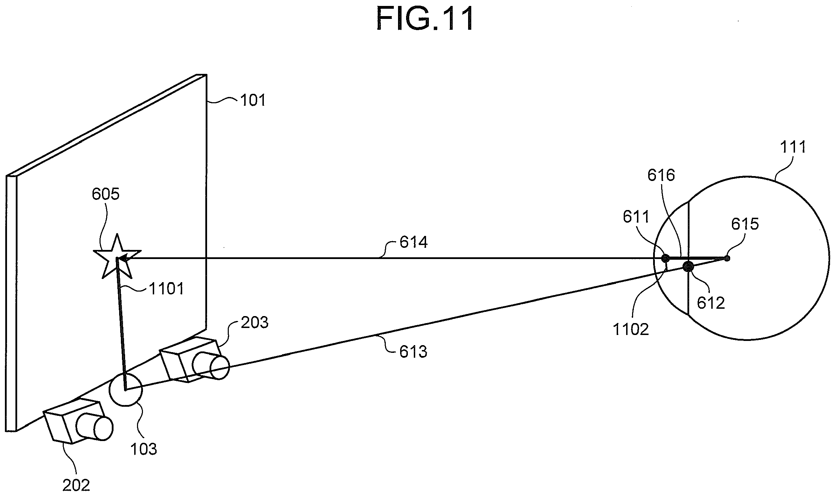

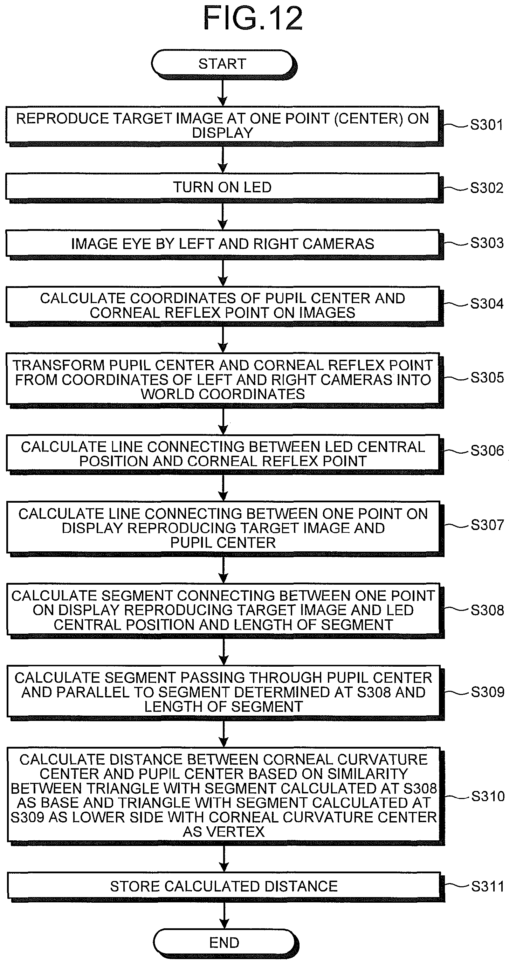

The calculation processing to calculate the distance between the pupil center position and the corneal curvature center position is not limited to the method described in FIG. 7 and FIG. 8. The following describes another example of the calculation processing with reference to FIG. 11 and FIG. 12.

FIG. 11 is a diagram for illustrating calculation processing of the present modification. The components described in FIG. 1 to FIG. 4 and FIG. 7 are attached with the same signs, and descriptions thereof are omitted.

A segment 1101 is a segment (a first segment) connecting between the target position 605 and the LED light source 103. A segment 1102 is a segment (a second segment) that is parallel to the segment 1101 and connects between the pupil center 611 and the line 613. The present modification calculates and stores the distance 616 between the pupil center 611 and the corneal curvature center 615 using the segment 1101 and the segment 1102 as follows.

FIG. 12 is a flowchart illustrating an example of the calculation processing of the present modification.

Steps S301 to S307 are similar to Steps S101 to S107 of FIG. 8, and descriptions thereof are omitted.

The third calculator 353 calculates a segment (the segment 1101 in FIG. 11) connecting between the center of the target image displayed at the one point of the screen of the display 101 and the center of the LED light source 103 and calculates the length (defined as L1101) of the calculated segment (Step S308).

The third calculator 353 calculates a segment (the segment 1102 in FIG. 11) that passes through the pupil center 611 and is parallel to the segment calculated at Step S308 and calculates the length (defined as L1102) of the calculated segment (Step S309).

The third calculator 353, based on a similarity relation between a triangle with the corneal curvature center 615 as a vertex and with the segment calculated at Step S308 as a base and a triangle with the corneal curvature center 615 as a vertex and with the segment calculated at Step S309 as a base, calculates the distance 616 between the pupil center 611 and the corneal curvature center 615 (Step S310). The third calculator 353, for example, calculates the distance 616 so that the ratio of the length of the segment 1102 to the length of the segment 1101 is equal to the ratio of the distance 616 to the distance between the target position 605 and the corneal curvature center 615.

The distance 616 can be calculated by the following equation (1), where L614 is the distance from the target position 605 to the pupil center 611.

$$\text{Distance } 616 = (L614 \times L1102)/(L1101 - L1102) \ldots (1)$$

The third calculator 353 stores the calculated distance 616 in the storage 150 or the like (Step S311). The stored distance is used for calculating the corneal curvature center at the time of subsequent visual point (visual line) detection.

The following describes details of diagnosis support processing. The present embodiment uses an image representing a cause of a certain event and the event as the diagnostic image. Measuring a dwell time at a gazing point for an area set in the diagnostic image can support diagnosis. This configuration can support diagnosis such as what point developmentally disabled people gaze at to obtain information in order to understand a causal relation, whether they are unable to understand the causal relation despite gazing at, or the like. In other words, diagnosis support with higher precision than conventional ones is provided.

FIG. 13 to FIG. 16 are diagrams illustrating examples of the diagnostic image for use in the present embodiment. Each of the diagnostic images of FIG. 13 to FIG. 16 is an example of an image indicating one scene contained in a continuous moving image. The continuous moving image may be a moving image containing a cut assignment (image switching or the like) in the middle thereof.

FIG. 13 is an image indicating a scene in which a person is walking on a road in front of a fence. A plurality of stones is at his feet. Areas are set for the image. The example of FIG. 13 sets an area M containing a person, an area H containing a head, an area C containing a stone (an example of a first object) as a cause by which the person falls down, and an area S containing a stone that is irrelevant to the event in which the person (an example of a second object) falls down. Each of the images of FIG. 13 to FIG. 16 has a coordinate system with the upper-left of the image as the point of origin (0, 0) and with the lower-right coordinates as (Xmax, Ymax).

FIG. 14 is an image at the moment when the person stumbles over the stone within the area C and is about to fall down. FIG. 15 is an image at the moment when the person stumbles over the stone within the area C and falls down. FIG. 16 is an image indicating a scene after the person stumbles over the stone within the area C and falls down. Areas similar to those of FIG. 13 are set also for the respective images of FIG. 14 to FIG. 16.

The present embodiment uses, as one of the diagnostic images, a still image obtained by capturing a part of the moving image before an event occurs or a still image equivalent to the still image and a still image obtained by capturing a part of the moving image after the event occurs or a still image equivalent to the still image.

In the examples of FIG. 13 to FIG. 16, the event is "falling down of the person." A still image 1 (FIG. 13) obtained by capturing a part of the moving image before the event and a still image 2 (FIG. 16) obtained by capturing a part of the moving image after the event are used as the diagnostic image. The number of the still images is not limited to two, and three or more still images may be used.

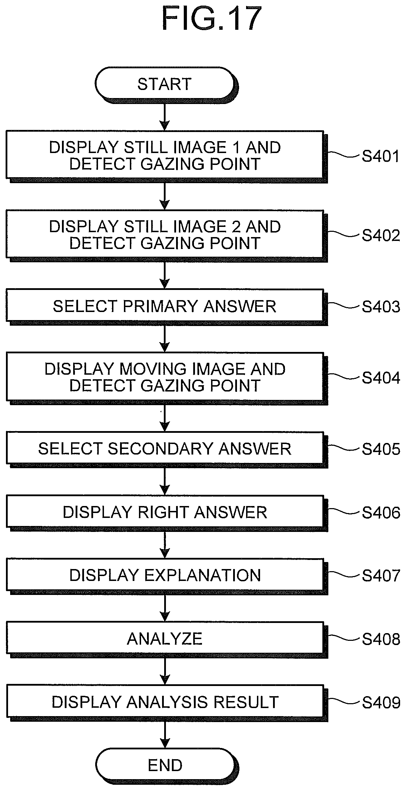

FIG. 17 is a flowchart illustrating an example of the diagnosis support processing when such diagnostic images are used.

First, the output controller 356 displays the still image 1 on the display 101. The subject sees the displayed still image 1. In this situation, the visual point detector 355 detects the gazing point (Step S401).

Next, the output controller 356 displays the still image 2 on the display 101. The subject sees the displayed still image 2. In this situation, the visual point detector 355 detects the gazing point (Step S402). Advancement from Step S401 to Step S402 may be performed in accordance with the pressing of a "proceed to next button" (not illustrated) or the like by the subject or an operator. The display may be continuously advanced without any instruction by the subject or the operator.

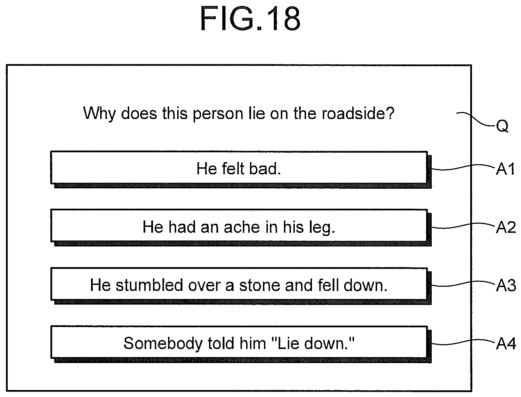

Next, the evaluator 357 receives a selection of a primary answer by the subject (Step S403). FIG. 18 is a diagram illustrating an example of a selection screen for selecting the primary answer. The primary answer is an answer selected after displaying the diagnostic images (the still image 1 and the still image 2). In the example of FIG. 18, the primary answer to a question Q is selected from among answer options A1 to A4. The answer options may include a noun indicating at least one of the cause and the event. In FIG. 18, for example, the option A3 to be a right answer contains a noun "stone" representing the cause and a verb "fell down" representing the event. Subjects having a developmental disorder have difficulty in determining a causal relation and often select any other than A3. On a probabilistic basis, the right answer may be selected even without understanding the causal relation. Only one question may therefore fail to support diagnosis with high precision. Consequently, inspections may be performed similarly for many kinds of videos. This operation can further improve the accuracy of diagnosis support.

The evaluator 357 acquires position information indicating a position touched by the subject or the operator from the display 101 configured as, for example, a touch panel and receives the selection of an option corresponding to the position information. The evaluator 357 may receive the primary answer designated using an input device (a keyboard or the like, not illustrated) by the subject or the operator. The selection of the primary answer is not limited to the method using the selection screen as illustrated in FIG. 18. For example, a method may be used that causes the operator to explain the question and the answer options orally and causes the subject to select the primary answer orally.

Returning back to FIG. 17, the output controller 356, for example, displays a moving image (a continuous moving image corresponding to the diagnostic images in FIG. 13 to FIG. 16) corresponding to the diagnostic images (the still image 1 and the still image 2) on the display 101. In this situation, the visual point detector 355 detects the gazing point (Step S404).

Details of gazing point detection processing at Step S401, Step S402, and Step S404 will be described below.

The evaluator 357 then receives a selection of a secondary answer by the subject (Step S405). The secondary answer is an answer selected after displaying the moving image corresponding to the diagnostic images. The selection of the secondary answer may be performed by, for example, a similar method to the selection of the primary answer. Options of the secondary answer may be the same as the options of the primary answer or different therefrom. The secondary answer is selected in order to enable the determination (the primary answer) after seeing the still image 1 and the still image 2 and the determination (the secondary answer) after seeing the continuous moving image subsequently to be compared with each other.

Next, the output controller 356 displays the right answer to the question on the display 101 (Step S406). The output controller 356 displays an explanation on the display 101 (Step S407).

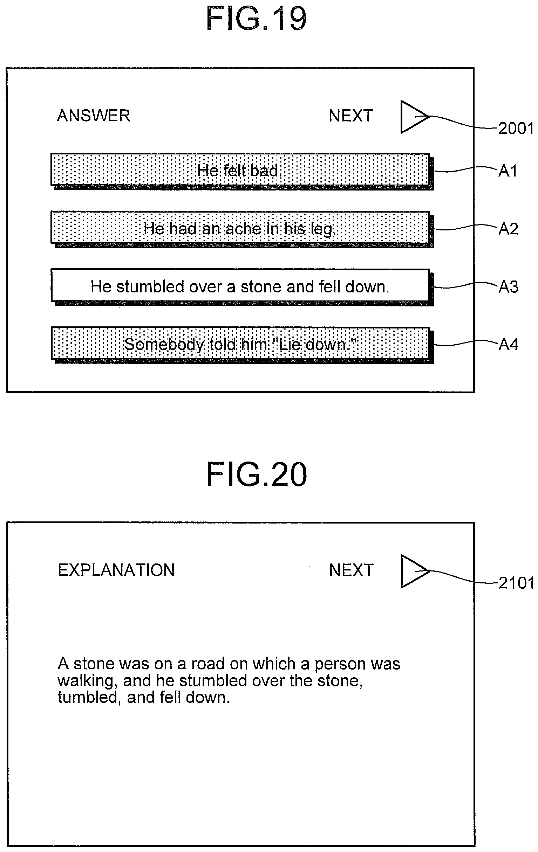

FIG. 19 is a diagram illustrating an example of a right answer screen for displaying the right answer. In the example of FIG. 19, the option A3 indicating the right answer is displayed in a display manner different from those of the other options. The method for indicating the right answer is not limited to this. If the options of the primary answer and the options of the secondary answer are different from each other, all the options may be displayed to highlight the option of the right answer.

On this right answer screen, for example, if a next button 2001 is pressed, an explanation screen for displaying an explanation is displayed. FIG. 20 is a diagram illustrating an example of the explanation screen. The explanation screen is displayed, thereby enabling the subject to understand the causal relation of the event indicated in the diagnostic images or the like. If a next button 2101 is pressed on the explanation screen, the display of the explanation screen is ended.

Returning back to FIG. 17, the evaluator 357 performs analysis processing based on data of the detected gazing point (Step S408). Details of the analysis processing will be described below. Finally, the output controller 356 displays an analysis result on the display 101 or the like (Step S409) .

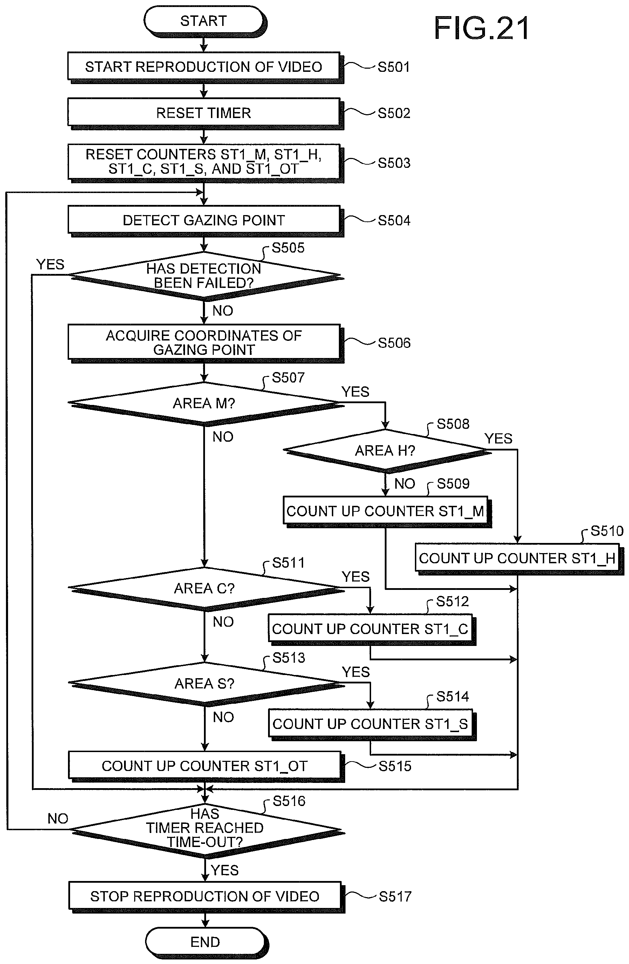

The following describes the details of the gazing point detection processing. FIG. 21 is a flowchart illustrating an example of the gazing point detection processing. FIG. 21 illustrates the gazing point detection processing after displaying the still image 1 at Step S401 of FIG. 17 as an example, and the pieces of gazing point detection processing at Step S402 (after displaying the still image 2) and Step S404 (after displaying the moving image) of FIG. 17 can also be achieved by a similar procedure.

First, the output controller 356 starts reproduction (display) of the diagnostic image (the still image 1) (Step S501). Next, the output controller 356 resets a timer for measuring a reproduction time (Step S502). Next, the visual point detector 355 resets counters (counters ST1_M, ST1_H, ST1_C, ST1_S, and ST1_OT) that count up at the time of gazing at the respective areas (Step S503).

The counters ST1_M, ST1_H, ST1_C, ST1_S, and ST1_OT are counters when the still image 1 (ST1) is displayed. The respective counters correspond to the following areas. By counting up the respective counters, dwell times representing times during which the gazing point is detected within the respective corresponding areas can be measured.

The counter ST1_M: the area M

The counter ST1_H: the area H

The counter ST1_C: the area C

The counter ST1_S: the area S

The counter ST1_OT: an area other than the above areas

Next, the visual point detector 355 detects the gazing point of the subject (Step S504). The visual point detector 355, for example, can detect the gazing point by the procedure described in FIG. 10. The visual point detector 355 determines whether detection of the gazing point has been failed (Step S505). When the images of the pupil and the corneal reflex cannot be obtained owing to a blink or the like, for example, the gazing point detection is failed. It may also be determined to be failed when the gazing point is absent within the display 101 (when the subject sees any other than the display 101).

If the detection of the gazing point has been failed (Yes at Step S505), the process advances to Step S516. If detection of the gazing point has been succeeded (No at Step S505), the visual point detector 355 acquires coordinates of the gazing point (gazing point coordinates) (Step S506).

The visual point detector 355 determines whether the acquired gazing point coordinates are present within the area M (around the person) (Step S507). If the acquired gazing point coordinates are present within the area M (Yes at Step S507), the visual point detector 355 further determines whether the acquired gazing point coordinates are present within the area H (around the head) (Step S508). If the acquired gazing point coordinates are present within the area H (Yes at Step S508), the visual point detector 355 increments (counts up) the counter ST1_H (Step S510), and the process proceeds to Step S516. If the acquired gazing point coordinates are absent within the area H (No at Step S508), the visual point detector 355 increments (counts up) the counter ST1_M (Step S509), and the process proceeds to Step S516.

If the acquired gazing point coordinates are absent within the area M (No at Step S507), the visual point detector 355 determines whether the acquired gazing point coordinates are present within the area C (around the object as the cause of the causal relation) (Step S511). If the acquired gazing point coordinates are present within the area C (Yes at Step S511), the visual point detector 355 increments (counts up) the counter ST1_C (Step S512), and the process proceeds to Step S516.

If the acquired gazing point coordinates are absent within the area C (No at Step S511), the visual point detector 355 determines whether the acquired gazing point coordinates are present within the area S (around the object that is not the cause of the causal relation) (Step S513). If the acquired gazing point coordinates are present within the area S (Yes at Step S513), the visual point detector 355 increments (counts up) the counter ST1_S (Step S514), and the process proceeds to Step S516.

If the acquired gazing point coordinates are absent within the area S (No at Step S513), the gazing point is absent within any of the set areas, and the visual point detector 355 increments (counts up) the counter ST1 OT (Step S515).

Next, the output controller 356 checks whether the timer that manages the reproduction time of the video has reached a time-out (Step S516). If a certain time has not been elapsed, that is, if the timer has not reached a time-out (No at Step S516), the process returns to Step S504 to continue the measurement. If the timer has reached a time-out (Yes at Step S516), the output controller 356 stops reproduction of the video (Step S517).

The gazing point detection processing when the still image 2 (ST2) at Step S402 is displayed can use a similar procedure to FIG. 21 by replacing the counters as follows:

The counter ST1_M → a counter ST2_M

The counter ST1_H → a counter ST2_H

The counter ST1_C → a counter ST2_C

The counter ST1_S → a counter ST2_S

The counter ST1_OT → a counter ST2_OT

The gazing point detection processing when the moving image (MOV) at Step S404 is displayed can use a similar procedure to FIG. 21 by replacing the counters as follows:

The counter ST1_M → a counter MOV_M

The counter ST1_H → a counter MOV_H

The counter ST1_C → a counter MOV_C

The counter ST1_S → a counter MOV_S

The counter ST1_OT → a counter MOV_OT

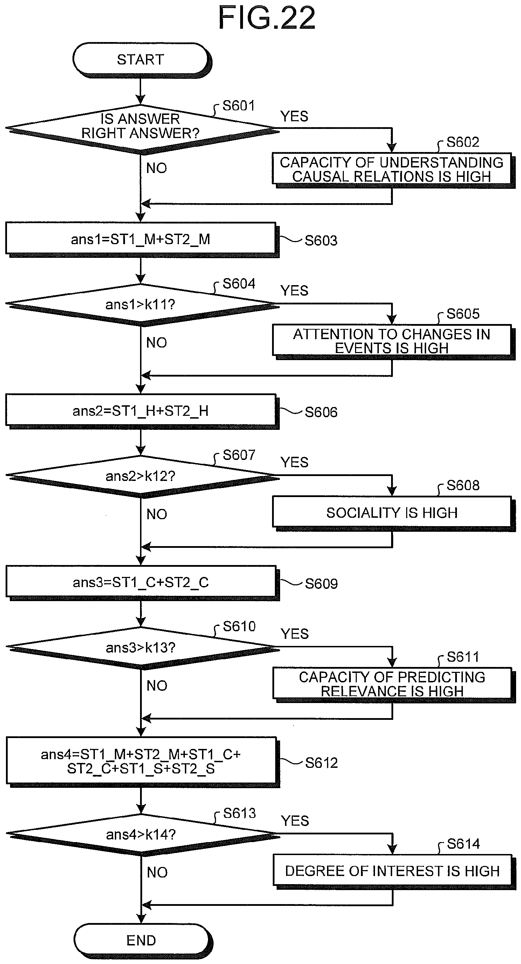

The following describes the details of the analysis processing. FIG. 22 is a flowchart illustrating an example of the analysis processing. The analysis processing and the evaluation value described below are examples and not limited to them. The evaluation value, for example, may be changed in accordance with the displayed diagnostic image.

First, the evaluator 357 determines whether the selected primary answer is the right answer (Step S601). If the selected primary answer is the right answer (Yes at Step S601), the evaluator 357 calculates an evaluation value indicating that capacity of understanding causal relations is high (Step S602).

If the primary answer is not the right answer (No at Step S601), or after Step S602, the evaluator 357 calculates ans1 = ST1_M + ST2_M (Step S603). ST1_M represents a value of the counter ST1_M, for example. The following may similarly represent a value of a counter X as simply "X."

Next, the evaluator 357 determines whether ans1 is larger than a threshold k11 (Step S604). If ans1 is larger than the threshold k11 (Yes at Step S604), the evaluator 357 calculates an evaluation value indicating that the degree of attention is high against changes in events (Step S605). This is because ans1 indicates a degree to which the gazing point is contained within the area M containing the person. The evaluation value may be binary values indicating that the degree of attention toward changes in events is high or low or multiple values varying in accordance with, for example, the magnitude of ans1.

If ans1 is equal to or less than the threshold k11 (No at Step S604), or after Step S605, the evaluator 357 calculates ans2 = ST1_H + ST2_H (Step S606). The evaluator 357 then determines whether ans2 is larger than a threshold k12 (Step S607). If ans2 is larger than the threshold k12 (Yes at Step S607), the evaluator 357 calculates an evaluation value indicating that the degree of attention toward the head containing one's face is high and that the development of sociality is high (Step S608). This is because ans2 indicates a degree to which the gazing point is contained within the area H containing the head.

If ans2 is equal to or less than the threshold k12 (No at Step S607), or after Step S608, the evaluator 357 calculates ans3 = ST1_C + ST2_C (Step S609). The evaluator 357 then determines whether ans3 is larger than a threshold k13 (Step S610). If ans3 is larger than the threshold k13 (Yes at S610), the evaluator 357 calculates an evaluation value indicating that capacity of predicting relevance is high and that attention to the object related to the causal relation is paid (Step S611). This is because ans3 indicates a degree to which the gazing point is contained within the area C containing the stone as the cause by which the person falls down.

If ans3 is equal to or less than the threshold k13 (No at Step S610), or after Step S611, the evaluator 357 calculates ans4 = ST1_M + ST2_M + ST1_C + ST2_C + ST1_S + ST2_S (Step S612). The evaluator 357 then determines whether ans4 is larger than a threshold k14 (Step S613). If ans4 is larger than the threshold k14 (Yes at Step S613), the evaluator 357 calculates an evaluation value, which indicates that the degree of interest toward various objects and events is high (Step S614). This is because ans4 indicates a degree to which the gazing point is contained within the areas (the area M, the area C, and the area S) containing the objects such as the person and the stone.

In the case that ans4 is equal to or less than the threshold k14 (No at Step S613), or after Step S614, the analysis processing ends. Similarly to ans1, ans2, ans3, and ans4 may be binary values or multiple values.

Subjects having a developmental disorder often have difficulty in understanding causal relations. It is desirable to change a method of care and education depending on whether a causal relation cannot be understood even though a subject gazes at a cause of the causal relation and incorporates its information into the brain or the causal relation cannot be understood because the subject does not try to see the cause of the causal relation, and information itself does not reach the brain. In particular, when a case has neither the evaluation value indicating that capacity of understanding causal relations is high (Step S602), the evaluation value indicating that the development of sociality is high (Step S608), nor the evaluation value indicating that capacity of predicting relevance is high (Step S611), the case has a possibility of a developmental disorder.

The diagnosis supporting device of the present embodiment, when images (the still image 1 and the still image 2, for example) before and after an event are displayed, measures what part a subject sees. With this configuration, diagnosis can be supported with high precision even about whether causal relations can be understood or the like. With an analyzed (diagnosed) result as reference, a policy of care and education can be determined.

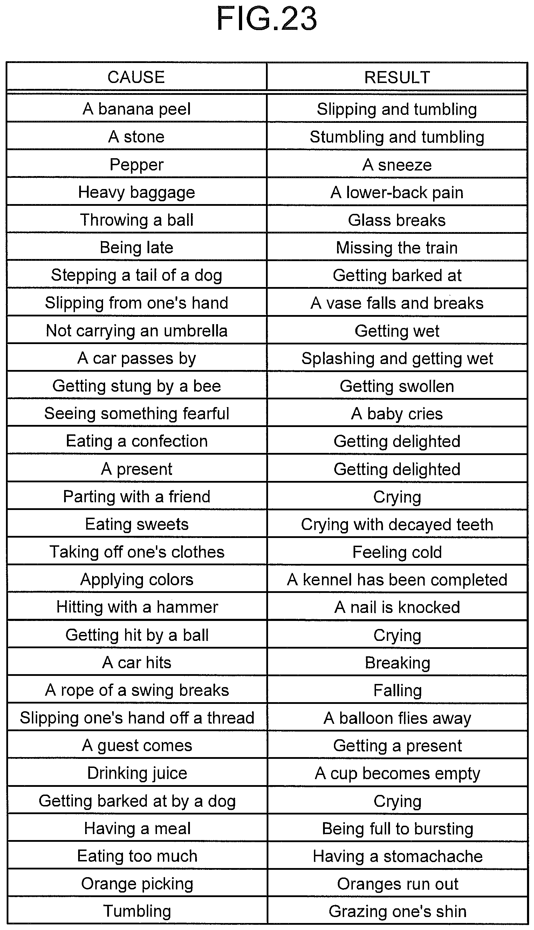

FIG. 23 is a diagram illustrating examples of the causal relation. It is illustrated that results described on the right column are produced in response to causes described on the left column. In place of the still image 1 and the still image 2, a still image indicating any cause described in FIG. 23 and a still image indicating a result corresponding to the cause may be used. Other than the examples of FIG. 23, various diagnostic images indicating causes and results can be used. The diagnostic images (two still images or the like) indicating causal relations as illustrated in FIG. 23, for example, may be displayed a plurality of times, and evaluation results for a plurality of diagnostic images may be integrated. For example, the values of the respective counters may not be reset each time the diagnostic images are displayed, and addition of the values of the counters for all the diagnostic images may be continued. In this case, the respective thresholds used in the analysis processing in FIG. 22, for example, may be changed in accordance with the number, type, or the like of the used diagnostic images. With this configuration, the accuracy of evaluation can further be increased.

As illustrated in FIG. 22, in principle, diagnosis is supported based on the gazing point when the images (the still image 1 and the still image 2) before and after the event are displayed. Display of the moving image, detection of the gazing point when the moving image is displayed, and display of the explanation (Step S404 to Step S407 in FIG. 17) are provided in order to tell the subject the right answer and enable evaluation on whether the subject has understood causal relations by seeing the moving image, or the like. These pieces of processing (Step S404 to Step S407 in FIG. 17) can be omitted for the purpose of diagnosis support alone, for example.

When the evaluation value (Step S611) indicating that capacity of predicting relevance is high is calculated, for example, diagnosis about understanding of causal relations can be supported. In this case, there is no need to display the right answer screen or to receive selection of the primary answer. This is because what is only needed is the detection result of the gazing point when the diagnostic image is displayed, for calculating the evaluation value as in Step S611.

In FIG. 22, the evaluation values were each independently calculated. Two or more of the conditions of FIG. 22 may be combined to determine an evaluation value. For example, If the primary answer is the right answer (Yes at Step S601) and if ans3 is larger than the threshold k13 (Yes at Step S610), the evaluation value indicating that capacity of predicting relevance is high may be calculated.

The diagnosis support processing of FIG. 17 includes display of the explanation image (moving image) illustrating the causal relation (Step S404), display of the right answer (Step S406), and display of the explanation (Step S407). Consequently, diagnosis can be supported, and in addition, support for training can also be achieved. Repeating the processing of FIG. 17 for the same diagnostic image or a plurality of different diagnostic images, for example, can provide more effective support for training.

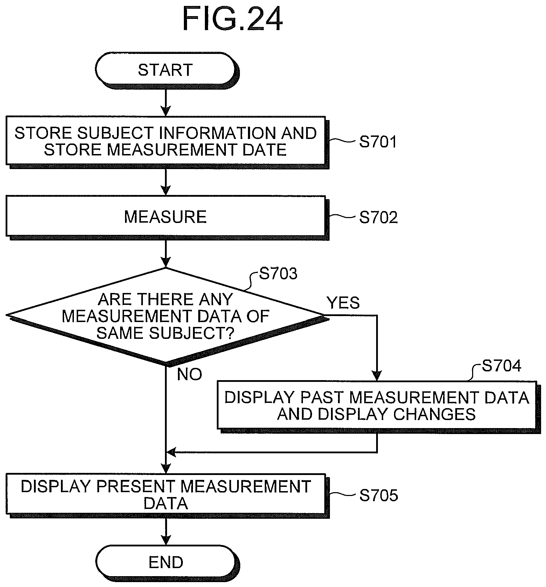

FIG. 24 is a flowchart illustrating an example of verification processing that verifies and displays effects of care and education.

First, the evaluator 357 stores subject information such as the name of a subject and a measurement date, for example, in the storage 150 before measurement (Step S701). Next, the diagnosis support processing (measurement) as illustrated in FIG. 17 is performed (Step S702). The evaluator 357 then determines whether past measurement data of the same subject is stored (Step S703). The measurement data includes, for example, values (dwell times) of the respective counters, ans1 to ans4 calculated from the values of the respective counters, and a part of or the entire evaluation values.

If the past measurement data is stored (Yes at Step S703), the output controller 356 displays information indicating the past measurement data and a change in the present measurement data relative to the past measurement data on the display 101 (Step S704).

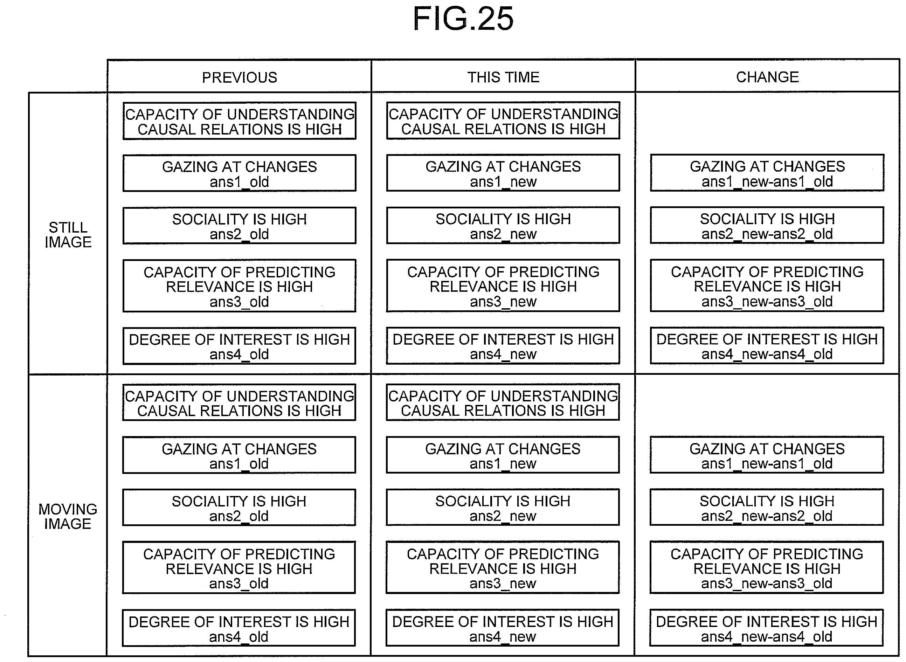

FIG. 25 is a diagram for illustrating an example of a method for determining changes in the measurement data. FIG. 25 illustrates examples in which changes in the present measurement data relative to the previous measurement data are separately determined for a still image and a moving image.

Concerning the evaluation value of "capacity of understanding causal relations is high," for example, it is illustrated that there is no change. ansn_old (n is 1 to 4) indicates values of the previous measurement data. ansn_new (n is 1 to 4) indicates values of the present measurement data. As illustrated in FIG. 25, changes in the measurement data can be determined by, for example, the difference between ansn_new and ansn_old.

The output controller 356 displays information (values indicating the difference) indicating thus determined changes in the measurement data, for example, on the display 101. The output controller 356 may output the measurement data and the information indicating changes to another device (an external communication device connected via a network, a printer, or the like) in place of the display 101.

Returning back to FIG. 24, in the case that the past measurement data is not stored (No at Step S703), or after the display processing at Step S704, the output controller 356 displays the present measurement data on the display 101 or the like (Step S705). If the past measurement data is present, the output controller 356 may simultaneously display the previous measurement data, the present measurement data, and the information indicating changes.

Thus, training by seeing the diagnostic image several times can increase the capacity of understanding causal relations and enables effects by the training to be checked.

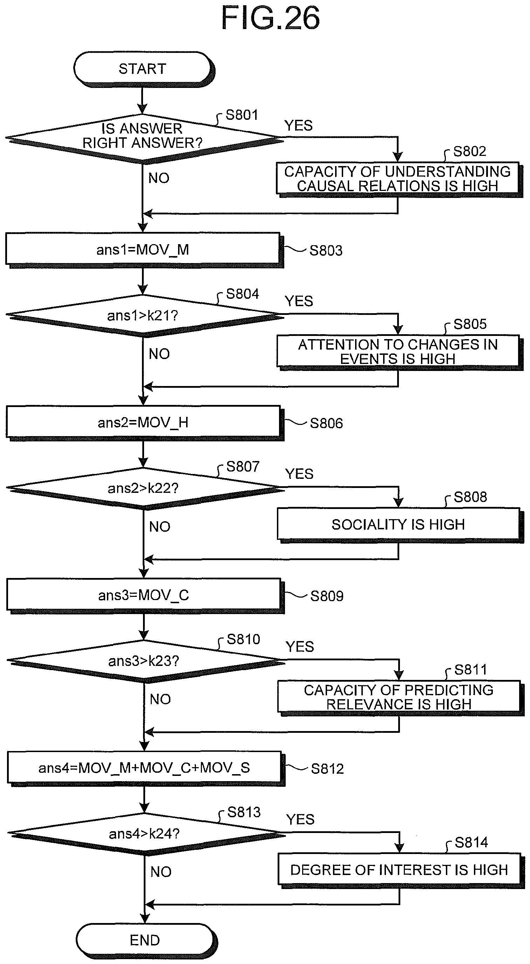

The analysis processing may be performed also when a moving image is displayed. FIG. 26 is a flowchart illustrating an example of the analysis processing when the moving image is displayed. In comparison with FIG. 22, in FIG. 26, the method for calculating ans1 to ans4 and the thresholds are changed as follows. The other procedure of processing is the same as that of FIG. 22, and a description thereof is omitted.

ans1 = MOV_M

ans2 = MOV_H

ans3 = MOV_C

ans4 = MOV_M + MOV_C + MOV_S

k11 → k21

k12 → k22

k13 → k23

k14 → k24

The analysis processing as illustrated in FIG. 26 enables the subject to be evaluated on an increase of understanding by seeing the moving image. For example, the evaluation result of the moving image may further be added to the evaluation result of FIG. 22. With this configuration, the accuracy of evaluation can further be increased.

In order to support the determination of the policy of care and education, methods of training (a policy of care and education) to be recommended may be displayed on the display 101 or the like in accordance with a diagnostic result. For example, the evaluator 357 may compare the measurement data with a threshold for policy determination determined in advance, and the output controller 356 may display different methods of training for a case of being lower than the threshold and a case of being the threshold or more. The output controller 356 may display different methods of training in accordance with a combination of values of different pieces of measurement data (the evaluation value indicating capacity of understanding causal relations is high and the evaluation value indicating capacity of predicting relevance is high, for example) or the like. The method of training may be a method of training using the present diagnosis supporting device 100, a method of training using illustrations and photographs, and any other method of training.

Although the above example uses the moving image containing the diagnostic images (the still image 1 and the still image 2) used at the time of diagnosis as the explanation image, the explanation image is not limited to such a moving image. Any image can be used so long as it is an image that represents a causal relation between a cause and an event and serves as support for training. For example, the explanation image containing one or more still images different from the diagnostic images may be used.

Modification 2

The above embodiment describes an example in which the diagnosis supporting device that supports diagnosis of a developmental disorder or the like is used also as a training supporting device. Any device that can display, for example, an explanation image, a right answer, an explanation, or the like other than the diagnosis supporting device can be used as the training supporting device. The present modification describes an example in which a portable terminal such as a tablet, a smartphone, and a notebook personal computer (PC) is used as the training supporting device. Other than that, an information processing device such as an ordinary personal computer can be used as the training supporting device.

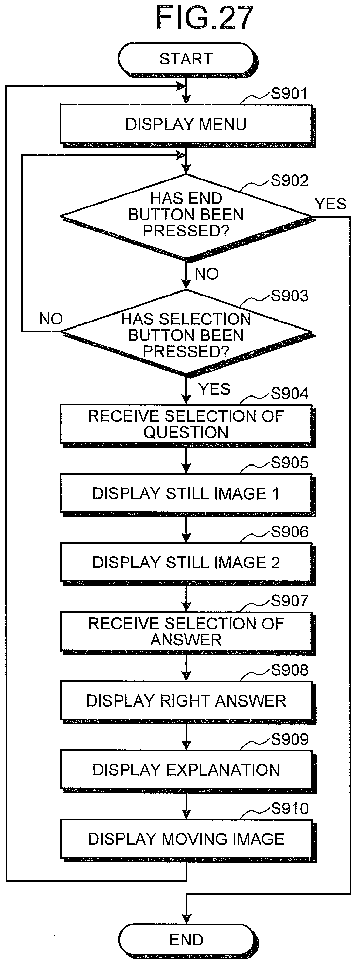

The gazing point detection processing performed at Step S401, Step S402, and Step S404 of the diagnosis support processing of FIG. 17, for example, is used for calculating the evaluation values used for diagnosis support mainly for a developmental disorder. When the object is training support, therefore, it is not necessary to perform the gazing point detection processing. The following describes an example of training support processing that does not include the gazing point detection processing. FIG. 27 is a flowchart illustrating an example of the training support processing of the present modification.

When a program for training support is started, for example, the output controller 356 displays a menu screen (Step S901).

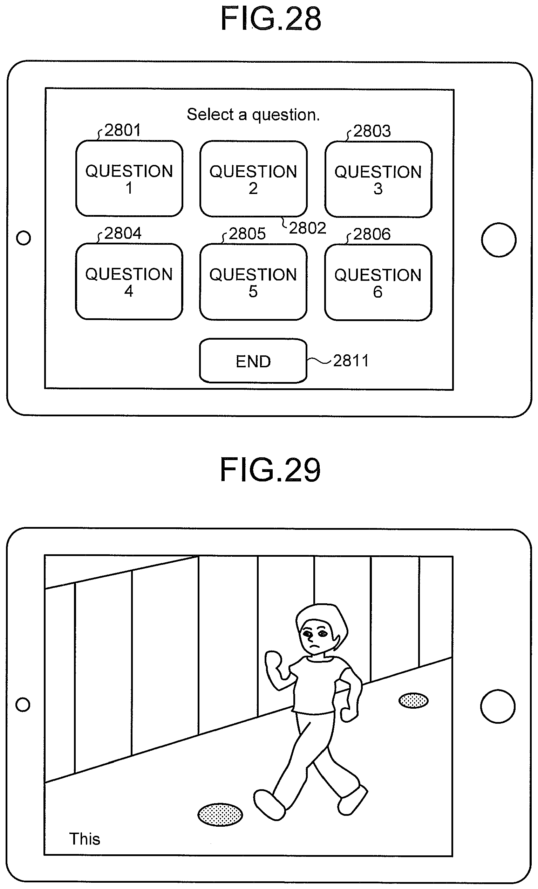

FIG. 28 is a diagram illustrating an example of the menu screen of Modification 2. As illustrated in FIG. 28, the menu screen contains selection buttons 2801 to 2806 for selecting a question and an end button 2811. If any of the selection buttons 2801 to 2806 is pressed, an image of a corresponding question is displayed. FIG. 28 illustrates an example in which six questions (Question 1 to Question 6) can be selected. The number of the questions and the method for selecting a question are not limited to the example of FIG. 28. If the end button 2811 is pressed, the program ends.

Returning back to FIG. 27, the output controller 356 determines whether the end button 2811 has been pressed (Step S902). If the end button 2811 has been pressed (Yes at Step S902), the output controller 356 ends the training support processing.

If the end button 2811 has not been pressed (No at Step S902), the output controller 356 determines whether any of the selection buttons 2801 to 2806 has been pressed (Step S903). If any of the selection buttons 2801 to 2806 has not been pressed (No at Step S903), the process returns to Step S902 to repeat the processing.

If any of the selection buttons 2801 to 2806 has been pressed (Yes at Step S903), the output controller 356 receives selection of a question corresponding to the pressed button among the selection buttons 2801 to 2806 (Step S904). The output controller 356 displays the still image 1 indicating the cause of the event among the images corresponding to the received question (Step S905). Suppose that, for example, a user (trainee) has pressed the selection button 2801 of FIG. 28 to select Question 1. FIG. 29 is a diagram illustrating an example of the still image 1 displayed in this situation. FIG. 29 illustrates an example of the still image containing an object (stone) as a cause.

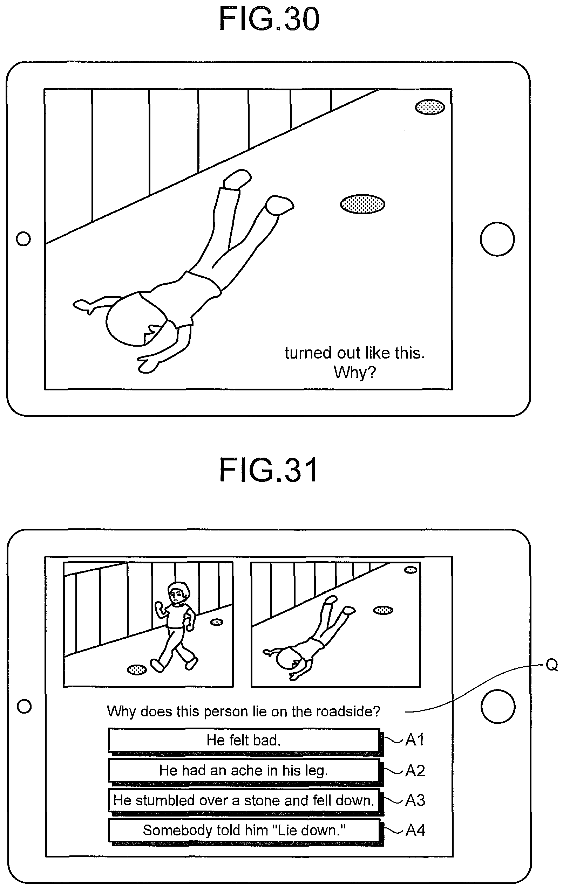

Returning back to FIG. 27, the output controller 356 displays the still image 1 for a certain time (10 seconds, for example) and then displays the still image 2 indicating an event for a certain time (10 seconds, for example) (Step S906). The display times of the respective still images may be the same or different from each other. FIG. 30 is a diagram illustrating an example of the still image 2 displayed in this situation. FIG. 30 is an example of the still image indicating a result (falling down) caused by the object (stone) as the cause.

Next, the output controller 356 receives selection of an answer (Step S907). FIG. 31 is a diagram illustrating an example of a selection screen for selecting the answer. FIG. 31 illustrates an example of the selection screen containing, together with the two still images (the still image 1 and the still image 2), a question Q and answer options A1 to A4. The user selects an answer to the question Q from among the answer options A1 to A4. The output controller 356 receives the answer thus selected by the user.

Returning back to FIG. 27, the output controller 356 displays a right answer to the question on the display 101 (Step S908). The output controller 356 displays an explanation on the display 101 (Step S909).

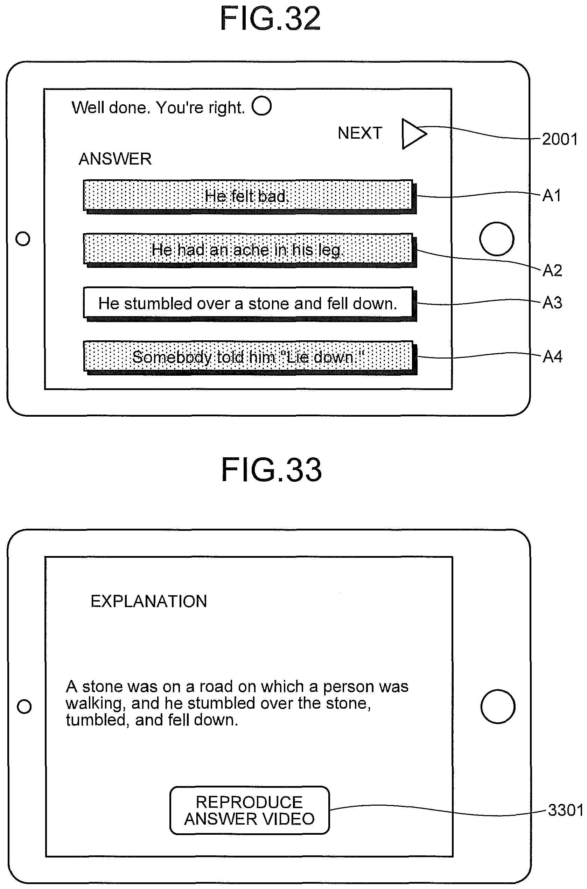

FIG. 32 is a diagram illustrating an example of a right answer screen for displaying the right answer. The example of FIG. 32 displays, together with a result of the answer ("Well done. You're right. O"), the option A3 indicating the right answer in a display manner (not grayed out) different from those of the other options. If a next button 2001 is pressed on this right answer screen, for example, an explanation screen for displaying an explanation is displayed. FIG. 33 is a diagram illustrating an example of the explanation screen. The explanation screen is displayed, thereby enabling the subject to understand the causal relation of the event or the like displayed in the diagnostic image. If a button 3301 is pressed on the explanation screen, a reproduction screen that reproduces a moving image (a reproduction video) is displayed.

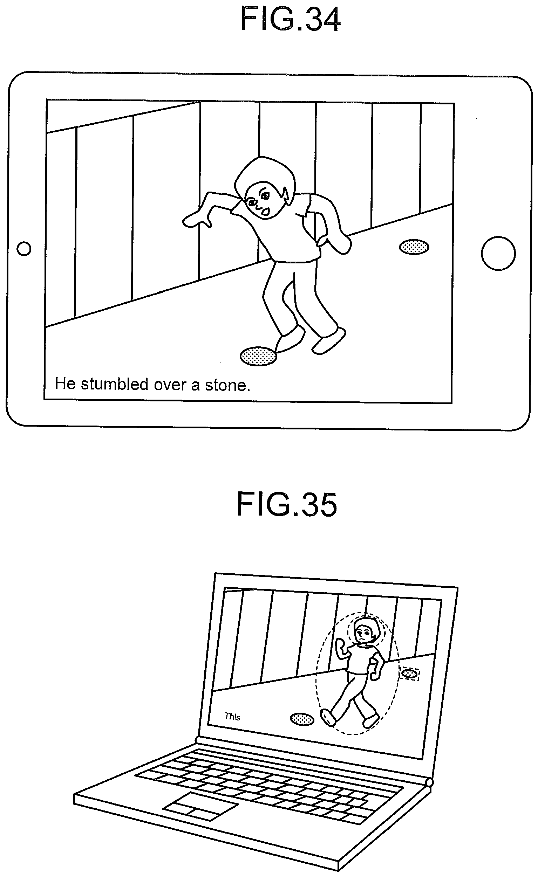

If the button 3301 is pressed on the explanation screen, the output controller 356 displays the reproduction screen on the display 101 (Step S910). The reproduction screen displays a moving image containing, for example, a process from the still image 1 to the still image 2. FIG. 34 is a diagram illustrating an example of the reproduction screen. This reproduction screen is an image at a certain point of time of the reproduced moving image and illustrates an example in which the image containing an explanation ("He stumbled over a stone") is displayed. Such a moving image is displayed, thereby enabling the user to further deepen understanding of the causal relation of the event or the like.

When the display of the reproduction screen ends, the process returns to the menu display (Step S901).

Such processing enables training even by an device such as a tablet, which does not incorporate a gazing point detector and is less expensive. It is noted that a doctor or the like cannot perform evaluation or guidance based on a gazing point during training.

FIG. 35 is a diagram illustrating an example of implementing a training supporting device by a notebook PC. FIG. 35 illustrates an example in which the still image 1 corresponding to FIG. 29 is displayed on a display (corresponding to the display 101) of the notebook PC.

If the answer by the user is the right answer, points or the like may be given for each right answer. With this configuration, the user is motivated to perform training, and training can be supported more effectively.

As described above, the present embodiment produces the following advantageous effects, for example.

(1) By causing a subject to view images for training multiple times, the subject is enabled to understand a causal relation effectively.
(2) Effects of training can be measured.
(3) Whether a subject gazes at an object related to a causal relation can be known.
(4) Points and directions of care and education can be set.
(5) Self-analysis is enabled.
(6) Development of sociality can also be checked.

(7) Without the need to arrange light sources (the illuminator) at two sites, visual line detection is enabled using a light source arranged at one site.
(8) Owing to the light source arranged at one site, the device can be compact, and cost reduction can also be achieved.

The diagnosis supporting device, the diagnosis supporting method, and the computer-readable recording medium according to the present embodiment produce the advantageous effect of increasing the accuracy of diagnosis.

Although the invention has been described with respect to specific embodiments for a complete and clear disclosure, the appended claims are not to be thus limited but are to be construed as embodying all modifications and alternative constructions that may occur to one skilled in the art that fairly fall within the basic teaching herein set forth.

## Claims

1.  A diagnosis supporting device (100) for developmental disorder comprising:

    a display (101);
    an imaging unit (102) configured to image a subject;
    a visual line detector (354) configured to detect a visual line direction of the subject from an image imaged by the imaging unit (102);
    a visual point detector (355) configured to detect a visual point of the subject in a display area of the display (101) based on the visual line direction;
    an output controller (356) configured to
    display a diagnostic image comprising an image representing a cause of a certain event before the event has taken place and an image representing a result corresponding to the cause after the event has taken place which indicate a causal relation in the order of the image representing the cause and the image representing the result on the display (101) ;
    display a question about the event on the display (101); and
    display a moving image corresponding to the image representing the cause and the image representing the result on the display (101) after displaying the diagnostic image on the display (101),
    an evaluator (357) configured to
    receive a selection of a primary answer selected among options displayed on the display (101) by the subject after displaying the diagnostic image on the display (101); and
    receive a selection of a secondary answer selected among options displayed on the display (101) by the subject after displaying the moving image on the display (101), wherein
    the output controller (356) is further configured to
    display a right answer and an explanation screen representing a causal relation between the image representing the cause and the image representing the result on the display (101) in the order of the right answer and the explanation screen after the evaluator (357) receives the selection of the secondary answer, wherein
    the evaluator (357) is further configured to calculate an evaluation value of the subject after receiving the selection of the secondary answer based on the respective visual point detected by the visual point detector when the diagnostic image is displayed on the display (101) and at least one of the primary answer and the secondary answer, and allow the output controller (356) to display the evaluation value on the display (101) after the output controller (356) displays the right answer and the explanation screen on the display (101).

2.  The diagnosis supporting device (100) for developmental disorder according to claim 1 wherein the evaluator (357) is configured to calculate the evaluation value based on at least one of a first dwell time indicating a time during which the visual point is detected in a first area containing a first object as the cause among areas in the image representing the cause contained in the diagnostic image and a second dwell time indicating a time during which the visual point is detected in a second area containing a second object as the result among the areas in the image representing the result contained in the diagnostic image.

3.  The diagnosis supporting device (100) for developmental disorder according to claim 2, wherein the evaluator (357) is configured to calculate the evaluation value further based on a third dwell time indicating a time during which the visual point is detected in an area other than the first area and the second area among the areas contained in the diagnostic image.

4.  The diagnosis supporting device (100) for developmental disorder according to any one of claims 1 to 3, wherein the output controller (356) is configured to display a plurality of diagnostic images, and

the evaluator (357) is configured to calculate the evaluation value of the subject based on the visual point detected by the visual point detector (355) when the plurality of diagnostic images are displayed.

5. The diagnosis supporting device (100) for developmental disorder according to any one of claims 1 to 4, further comprising:

an illuminator including a light source (103) that emits light;
a position detector configured to detect a first position indicating center of a pupil and a second position indicating center of a corneal reflex from an image of an eyeball of the subject to which light is applied by the illuminator and that is imaged by the imaging unit; and
a calculator configured to calculate a fourth position indicating a corneal curvature center based on a position of the light source, a third position on the display, the first position, and the second position, wherein
the visual line detector (354) is configured to detect a visual line of the subject based on the first position and the fourth position.

6. A diagnosis supporting method for developmental disorder comprising:

detecting, from an image imaged by an imaging unit (102) that images a subject, a visual line direction of the subject by a visual line detector (354);
detecting a visual point of the subject in a display area of a display (101) based on the visual line direction;
displaying a diagnostic image comprising an image representing a cause of a certain event before the event has taken place and an image representing a result corresponding to the cause after the event has taken place which indicate a causal relation in the order of the image representing the cause and the image representing the result on the display (101) ;
displaying a question about the event on the display (101) ;
receiving a selection of a primary answer selected among options displayed on the display (101) by the subject after displaying the diagnostic image on the display (101);
displaying a moving image corresponding to the image representing the cause and the image representing the result on the display (101) after receiving the selection of the primary answer;
receiving a selection of a secondary answer selected among options displayed on the display (101) by the subject after displaying the moving image on the display (101);
displaying a right answer and an explanation screen representing a causal relation between the image representing the cause and the image representing the result on the display (101) in the order of the right answer and the explanation screen after receiving the selection of the secondary answer;
calculating an evaluation value of the subject after receiving the selection of the secondary answer based on the respective visual point detected at the detecting of the visual point of the subject when the diagnostic image is displayed on the display (101) and at least one of the primary answer and the secondary answer; and
displaying the evaluation value on the display (101) after displaying the right answer and the explanation screen on the display (101).

7. A non-transitory computer-readable recording medium that therein stores a computer program causing a computer to execute a diagnosis supporting method for developmental disorder , the method comprising:

detecting, from an image imaged by an imaging unit (102) that images a subject, a visual line direction of the subject by a visual line detector (354);
detecting a visual point of the subject in a display area of a display (101) based on the detected visual line direction;
displaying a diagnostic image comprising an image representing a cause of a certain event before the event has taken place and an image representing a result corresponding to the cause after the event has taken place which indicate a causal relation in the order of the image representing the cause and the image representing the result on the display (101);
displaying a question about the event on the display (101);
receiving a selection of a primary answer selected among options displayed on the display (101) by the subject after displaying the diagnostic image on the display (101);
displaying a moving image corresponding to the image representing the cause and the image representing the result on the display (101) after receiving the selection of the primary answer;
receiving a selection of a secondary answer selected among options displayed on the display (101) by the subject after displaying the moving image on the display (101);
displaying a right answer and an explanation screen representing a causal relation between the t image representing the cause and the image representing the result on the display (101) in the order of the right answer and the explanation screen after receiving the selection of the secondary answer;

calculating an evaluation value of the subject after receiving the selection of the secondary answer based on the detected respective visual point when the diagnostic image is displayed on the display (101) and at least one of the primary answer and the secondary answer; and

displaying the evaluation value on the display (101) after displaying the right answer and the explanation screen on the display (101).

**Patentansprüche**

1. Diagnoseunterstützungsvorrichtung (100) für eine Entwicklungsstörung, die Folgendes aufweist:

eine Anzeige (101);

eine Abbildungseinheit (102), die konfiguriert ist zum Abbilden eines Subjekts;

einen Blickliniendetektor (354), der konfiguriert ist zum Detektieren einer Blicklinienrichtung des Subjekts von einem Bild, das von der Abbildungseinheit (102) abgebildet wird;

einen Blickpunktdetektor (355), der konfiguriert ist zum Detektieren eines Blickpunktes des Subjekts in einem Anzeigebereich der Anzeige (101) basierend auf der Blicklinienrichtung;

eine Ausgangsteuerung (356), die konfiguriert ist zum Anzeigen eines Diagnosebildes, das ein Bild aufweist, das eine Ursache eines bestimmten Ereignisses darstellt, bevor das Ereignis stattgefunden hat, und ein Bild aufweist, das ein Ergebnis darstellt, das mit der Ursache korrespondiert, nachdem das Ereignis stattgefunden hat, welches eine kausale Beziehung in der Reihenfolge des Bildes, das die Ursache darstellt anzeigt, und das Bild das Ergebnis auf der Anzeige (101) darstellt;

Anzeigen einer Frage über das Ereignis auf der Anzeige (101); und

Anzeigen eines bewegten Bildes, das mit dem Bild korrespondiert, das die Ursache anzeigt, und dem Bild, das das Ergebnis anzeigt auf der Anzeige (101), nach Anzeigen des Diagnosebild auf der Anzeige (101),

einen Evaluator (357), der konfiguriert ist zum

Empfangen einer Auswahl einer primären Antworten, die durch das Subjekt unter Optionen ausgewählt wird, die auf der Anzeige (101) angezeigt werden nach Anzeigen des Diagnosebildes auf der Anzeige (101); und

Empfangen einer Auswahl von sekundären Antworten, die durch das Subjekt unter den Optionen ausgewählt werden, die auf der Anzeige (101) angezeigt werden nach Anzeigen des bewegten Bildes auf der Anzeige (101), wobei

die Ausgangssteuerung (356) ferner konfiguriert ist zum Anzeigen einer richtigen Antwort, und eines Erklärungsbildschirms, der eine kausale Beziehung zwischen dem Bild anzeigt, das die Ursache anzeigt und dem Bild, das das Ergebnis auf der Anzeige (101) in der Reihenfolge der richtigen Antwort und des Erklärungsbildschirms anzeigt, nachdem der Evaluator (357) die Auswahl der sekundären Antworten empfängt, wobei

der Evaluator (357) ferner konfiguriert ist zum Berechnen eines Evaluationswertes des Subjekts nach Empfangen der Auswahl der sekundären Antwort basierend auf dem jeweiligen Blickpunkt, der durch den Blickpunktdetektor detektiert wird, wenn das Diagnosebild auf der Anzeige (101) angezeigt wird, und der primären Antwort und/oder der sekundären Antwort, und um der Ausgangssteuerung (356) zu erlauben, den Evaluationswert auf der Anzeige (101) anzuzeigen, nachdem die Ausgangssteuerung (356) die richtige Antwort und den Erklärungsbildschirm auf der Anzeige (101) anzeigt.

2. Diagnoseunterstützungsvorrichtung (100) für eine Entwicklungsstörung nach Anspruch 1, wobei der Evaluator (357) konfiguriert ist zum Berechnen des Evaluationswertes basierend auf wenigstens einer ersten Verweilzeit, die eine Zeit angibt, während der der Blickpunkt in einem ersten Bereich detektiert wird, der ein erstes Objekt enthält als die Ursache unter den Bereichen des die Ursache repräsentierenden Bildes in dem diagnostischen Bild und einer zweiten Verweilzeit, die eine Zeit angibt, während der der Blickpunkt in einem zweiten Bereich detektiert wird, der ein zweites Objekt als das Ereignis enthält unter den Bereichen des das Ergebnis repräsentierenden Bildes in dem diagnostischen Bild.

3. Diagnoseunterstützungsvorrichtung (100) für eine Entwicklungsstörung nach Anspruch 2, wobei der Evaluator (357) konfiguriert ist zum Berechnen des Evaluationswertes ferner basierend auf einer dritten Verweilzeit, die eine Zeit angibt, während der der Blickpunkt in einem anderen Bereich als dem ersten Bereich und dem zweiten Bereich unter den Bereichen, die in dem diagnostischen Bild enthalten sind, detektiert wird.

4. Diagnoseunterstützungsvorrichtung (100) für eine Entwicklungsstörung nach einem der Ansprüche 1 bis 3, wobei

die Ausgangssteuerung (356) konfiguriert ist zum Anzeigen einer Vielzahl von Diagnosebildern, und
der Evaluator (357) konfiguriert ist zum Berechnen des Berechnungswertes des Subjekts basierend auf dem Blickpunkt, der von dem Blickpunktdetektor (355) detektiert wird, wenn die Vielzahl von diagnostischen Bildern angezeigt wird.

5. Diagnoseunterstützungsvorrichtung (100) für eine Entwicklungsstörung nach einem der Ansprüche 1 bis 4, die ferner Folgendes aufweist:

eine Beleuchtungsvorrichtung mit einer Lichtquelle (103), die Licht emittiert;
einen Positionsdetektor, der konfiguriert ist zum Detektieren einer ersten Position, die das Zentrum einer Pupille anzeigt, und einer zweiten Position, die das Zentrum einer Hornhautreflektion von einem Bild eines Augapfels des Subjekts anzeigt, auf das Licht von der Beleuchtungsvorrichtung angelegt wird und das durch die Abbildungseinheit abgebildet wird; und
einen Rechner, der konfiguriert ist zum Berechnen einer vierten Position, die ein Hornhautkrümmungszentrum anzeigt basierend auf einer Position der Lichtquelle, einer dritten Position auf der Anzeige, der ersten Position und der zweiten Position, wobei
der Blickliniendetektor (354) konfiguriert ist zum Detektieren einer Blicklinie des Subjekts basierend auf der ersten Position und der vierten Position.

6. Diagnoseunterstützungsverfahren für eine Entwicklungsstörung, die Folgendes aufweist:

Detektieren von einem Bild, das durch eine Bildgebungseinheit (102), die ein Subjekt abbildet, abgebildet wird, einer Blicklinienrichtung des Subjekts durch einen Blickliniendetektor (354);
Detektieren eines Blickpunktes des Subjekts in einem Anzeigebereich einer Anzeige (101) basierend auf der Blicklinienrichtung;
Anzeigen eines Diagnosebildes, das ein Bild aufweist, das eine Ursache eines bestimmten Ereignisses darstellt, bevor das Ereignis stattgefunden hat, und ein Bild, das ein Ergebnis darstellt, das mit der Ursache korrespondiert nachdem das Ereignis stattgefunden hat, welches eine kausale Beziehung in der Reihenfolge des Bildes anzeigt, das die Ursache darstellt, und des Bildes, das das Ergebnis darstellt auf der Anzeige (101);
Anzeigen einer Frage über das Ereignis auf der Anzeige (101);
Empfangen einer Auswahl einer primären Antwort, die durch das Subjekt unter den Optionen ausgewählt wird, die auf der Anzeige (101) anzeigt werden nachdem das Diagnosebild auf der Anzeige (101) angezeigt wird;
Anzeigen eines bewegten Bildes, das mit dem Bild korrespondiert, dass die Ursache darstellt und dem Bild, das das Ergebnis darstellt auf der Anzeige (101), nachdem die Auswahl der primären Antwort empfangen wurde;
Empfangen einer Auswahl einer sekundären Antwort, die durch das Subjekt unter den Optionen ausgewählt wird, die auf der Anzeige (101) angezeigt werden, nachdem das bewegte Bild auf der Anzeige (101) angezeigt wurde;
Anzeigen einer richtigen Antwort und eines Erklärungsbildschirms, der eine kausale Beziehung zwischen dem Bild darstellt, das die Ursache darstellt, und dem Bild, das das Ergebnis darstellt auf der Anzeige (101) in der Reihenfolge der richtigen Antwort, und dem Erklärungsbildschirm nach Empfangen der Auswahl der sekundären Antwort;
Berechnen eines Evaluierungswerts des Subjekts nach Empfangen der Auswahl der sekundären Antwort basierend auf dem Blickpunkt, der bei der Detektion des Blickpunktes des Subjekts detektiert wird, wenn das diagnostische Bild auf der Anzeige (101) angezeigt wird und der primären Antwort und/oder der sekundären Antwort;
und Anzeigen des Evaluationswertes auf der Anzeige (101) nach Anzeigen der richtigen Antwort und des Erklärungsbildschirms auf der Anzeige (101).

7. Ein nicht-transitorisches computerlesbares Aufnahmemedium, das darin ein Computerprogramm speichert, dass einen Computer veranlasst, ein Diagnoseunterstützungsverfahren für eine Entwicklungsstörung auszuführen, wobei das Verfahren Folgendes aufweist:

Detektieren von einem Bild, das durch eine Abbildungseinheit (102) angezeigt wird, welche ein Subjekt abbildet, einer Blicklinienrichtung des Subjekts durch einen Blickliniendetektor;
Detektieren eines Blickpunktes des Subjekts in einem Anzeigenbereich einer Anzeige (101) basierend auf der detektierten Blicklinienrichtung;
Anzeigen eines Diagnosebildes, das ein Bild aufweist, das eine Ursache eines bestimmten Ereignisses darstellt, bevor das Ereignis stattgefunden hat, und ein Bild aufweist, das ein Ergebnis darstellt, das mit der Ursache korrespondiert, nachdem das Ereignis stattgefunden hat, welches eine kausale Beziehung in der Reihenfolge des Bildes, das die Ursache darstellt, und des Bildes, dass das Ergebnis darstellt, auf der Anzeige (101) anzeigt; Anzeigen einer Frage über das Ereignis auf der Anzeige (101); Empfangen einer Auswahl von einer primären Antwort, die durch das Subjekt unter den Optionen ausgewählt wird, die auf der Anzeige (101) angezeigt werden nach Anzeigen des diagnostischen Bildes auf der Anzeige (101); Anzeigen eines bewegten Bildes, das mit dem Bild korrespondiert, das die Ursache darstellt und dem Bild, das das Ergebnis darstellt auf der Anzeige (101) nach Empfangen der Auswahl von der sekundären Antwort; Empfangen einer Auswahl von einer sekundären Antwort, die durch das Subjekt unter den Optionen ausgewählt wird, die auf der Anzeige (101) angezeigt werden nach Anzeigen des bewegten Bildes auf der Anzeige (101); Anzeigen einer richtigen Antwort und eines Erklärungsbildschirms, der eine kausale Beziehung zwischen dem t-Bild darstellt, das die Ursache darstellt und dem Bild, dass das Ergebnis darstellt auf der Anzeige (101) in der Reihenfolge der richtigen Antwort und des Erklärungsbildschirms, nach Empfangen der Auswahl der zweiten Antwort; Berechnen eines Evaluationswertes des Subjekts nach Empfangen der Auswahl der sekundären Antwort basierend auf dem detektierten Blickpunkt, wenn das diagnostische Bild auf der Anzeige (101) dargestellt wird, und der primären Antwort und/oder der sekundären Antwort; und Anzeigen des Evaluationswertes auf der Anzeige (101) nach Anzeigen der richtigen Antwort und des Erklärungsbildschirms auf der Anzeige (101).

## Revendications

1. Dispositif d'aide au diagnostic (100) pour des troubles du développement, comprenant :

   un afficheur (101) ;
   un module d'imagerie (102) agencé pour capturer des images d'un sujet ;
   un détecteur de ligne visuelle (354) agencé pour détecter une direction de ligne visuelle du sujet à partir d'une image capturée par le module d'imagerie (102) ;
   un détecteur de point visuel (355) agencé pour détecter un point visuel du sujet dans une région d'affichage de l'afficheur (101) sur la base de la direction de ligne visuelle ;
   un contrôleur de sortie (356) agencé pour
   afficher une image de diagnostic comprenant une image représentant une cause d'un certain événement avant que l'événement ait lieu et une image représentant un résultat correspondant à la cause après que l'événement a eu lieu, qui indiquent une relation causale dans l'ordre de l'image représentant la cause et de l'image représentant le résultat sur l'afficheur (101) ;
   afficher une question au sujet de l'événement sur l'afficheur (101) ; et
   afficher une image mobile correspondant à l'image représentant la cause et à l'image représentant le résultat sur l'afficheur (101) après l'affichage de l'image de diagnostic sur l'afficheur (101),
   un évaluateur (357) agencé pour
   recevoir une sélection d'une réponse primaire sélectionnée parmi des options affichées sur l'afficheur (101) par le sujet après l'affichage de l'image de diagnostic sur l'afficheur (101) ; et
   recevoir une sélection d'une réponse secondaire sélectionnée parmi des options affichées sur l'afficheur (101) par le sujet après l'affichage de l'image mobile sur l'afficheur (101), dans lequel
   le contrôleur de sortie (356) est en outre agencé pour
   afficher une bonne réponse et un écran d'explication représentant une relation causale entre l'image représentant la cause et d'image représentant le résultat sur l'afficheur (101) dans l'ordre de la bonne réponse et de l'écran d'explication après que l'évaluateur (357) a reçu la sélection de la réponse secondaire, dans lequel l'évaluateur (357) est en outre agencé pour calculer une valeur d'évaluation du sujet après la réception de la sélection de la réponse secondaire sur la base du point visuel respectif détecté par le détecteur de point visuel lorsque l'image de diagnostic est affichée sur l'afficheur (101) et d'au moins l'une de la réponse primaire et de la réponse secondaire, et permettre au contrôleur de sortie (356) d'afficher la valeur d'évaluation sur l'afficheur (101) après que le contrôleur de sortie (356) a affiché la bonne réponse et l'écran d'explication sur l'afficheur (101) .

2. Dispositif d'aide au diagnostic (100) pour des troubles du développement selon la revendication 1, dans lequel l'évaluateur (357) est agencé pour calculer la valeur d'évaluation sur la base d'au moins l'un d'un premier temps de maintien indiquant un temps pendant lequel le point visuel est détecté dans une première région contenant un premier objet en tant que cause parmi des régions dans l'image représentant la cause contenue dans l'image de diagnostic, et un deuxième temps de maintien indiquant un temps pendant lequel le point visuel est détecté dans une deuxième région contenant un deuxième objet en tant que résultat parmi les régions dans l'image représentant le résultat contenue dans l'image de diagnostic.

3. Dispositif d'aide au diagnostic (100) pour des troubles du développement selon la revendication 2, dans lequel l'évaluateur (357) est agencé pour calculer la valeur d'évaluation en outre sur la base d'un troisième temps de maintien indiquant un temps pendant lequel le point visuel est détecté dans une région autre que la première région et la deuxième région parmi les régions contenues dans l'image de diagnostic.

4. Dispositif d'aide au diagnostic (100) pour des troubles du développement selon l'une quelconque des revendications 1 à 3, dans lequel
le contrôleur de sortie (356) est agencé pour afficher une pluralité d'images de diagnostic, et
l'évaluateur (357) est agencé pour calculer la valeur d'évaluation du sujet sur la base du point visuel détecté par le détecteur de point visuel (355) lorsque la pluralité d'images de diagnostic est affichée.

5. Dispositif d'aide au diagnostic (100) pour des troubles du développement selon l'une quelconque des revendications 1 à 4, comprenant en outre :

un dispositif d'éclairage comprenant une source de lumière (103) qui émet de la lumière ;
un détecteur de position agencé pour détecter une première position indiquant le centre d'une pupille et une deuxième position indiquant le centre d'une réflexion cornéenne à partir d'une image d'un globe oculaire du sujet auquel de la lumière est appliquée par le dispositif d'éclairage et qui est capturée par le module d'imagerie ; et
un calculateur agencé pour calculer une quatrième position indiquant un centre de courbure cornéenne sur la base d'une position de la source de lumière, d'une troisième position sur l'afficheur, de la première position et de la deuxième position, dans lequel
le détecteur de ligne visuelle (354) est agencé pour détecter une ligne visuelle du sujet sur la base de la première position et de la quatrième position.

6. Procédé d'aide au diagnostic pour des troubles du développement, comprenant :

détecter, à partir d'une image capturée par un module d'imagerie (102) qui capture des images d'un sujet, une direction de ligne visuelle du sujet par un détecteur de ligne visuelle (354) ;
détecter un point visuel du sujet dans une région d'affichage d'un afficheur (101) sur la base de la direction de ligne visuelle ;
afficher une image de diagnostic comprenant une image représentant une cause d'un certain événement avant que l'événement ait lieu et une image représentant un résultat correspondant à la cause après que l'événement a eu lieu, qui indiquent une relation causale dans l'ordre de l'image représentant la cause et l'image représentant le résultat sur l'afficheur (101) ;
afficher une question au sujet de l'événement sur l'afficheur (101) ;
recevoir une sélection d'une réponse primaire sélectionnée parmi des options affichées sur l'afficheur (101) par le sujet après l'affichage de l'image de diagnostic sur l'afficheur (101) ;
afficher une image mobile correspondant à l'image représentant la cause et à l'image représentant le résultat sur l'afficheur (101) après la réception de la sélection de la réponse primaire ;
recevoir une sélection d'une réponse secondaire sélectionnée parmi des options affichées sur l'afficheur (101) par le sujet après l'affichage de l'image mobile sur l'afficheur (101) ;
afficher une bonne réponse et un écran d'explication représentant une relation causale entre l'image représentant la cause et l'image représentant le résultat sur l'afficheur (101) dans l'ordre de la bonne réponse et de l'écran d'explication après la réception de la sélection de la réponse secondaire ;
calculer une valeur d'évaluation du sujet après la réception de la sélection de la réponse secondaire sur la base du point visuel respectif détecté à la détection du point visuel du sujet lorsque l'image de diagnostic est affichée sur l'afficheur (101) et d'au moins l'une de la réponse primaire et de la réponse secondaire ; et
afficher la valeur d'évaluation sur l'afficheur (101) après l'affichage de la bonne réponse et de l'écran d'explication sur l'afficheur (101).

7. Support d'enregistrement non transitoire lisible par un ordinateur qui mémorise un programme d'ordinateur amenant un ordinateur à exécuter un procédé d'aide au diagnostic pour des troubles du développement, le procédé comprenant :

détecter, à partir d'une image capturée par un module d'imagerie (102) qui capture l'image d'un sujet, une direction de ligne visuelle du sujet par un détecteur de ligne visuelle (354) ;

détecter un point visuel du sujet dans une région d'affichage d'un afficheur (101) sur la base de la direction de ligne visuelle ;

afficher une image de diagnostic comprenant une image représentant une cause d'un certain événement avant que l'événement ait lieu et une image représentant un résultat correspondant à la cause après que l'événement a eu lieu, qui indiquent une relation causale dans l'ordre de l'image représentant la cause et l'image représentant le résultat sur l'afficheur (101) ;

afficher une question au sujet de l'événement sur l'afficheur (101) ;

recevoir une sélection d'une réponse primaire sélectionnée parmi des options affichées sur l'afficheur (101) par le sujet après l'affichage de l'image de diagnostic sur l'afficheur (101) ;

afficher une image mobile correspondant à l'image représentant la cause et à l'image représentant le résultat sur l'afficheur (101) après la réception de la sélection de la réponse primaire ;

recevoir une sélection d'une réponse secondaire sélectionnée parmi des options affichées sur l'afficheur (101) par le sujet après l'affichage de l'image mobile sur l'afficheur (101) ;

afficher une bonne réponse et un écran d'explication représentant une relation causale entre l'image représentant la cause et l'image représentant le résultat sur l'afficheur (101) dans l'ordre de la bonne réponse et de l'écran d'explication après la réception de la sélection de la réponse secondaire ;

calculer une valeur d'évaluation du sujet après la réception de la sélection de la réponse secondaire sur la base du point visuel respectif détecté lorsque l'image de diagnostic est affichée sur l'afficheur (101) et d'au moins l'une de la réponse primaire et de la réponse secondaire ; et

afficher la valeur d'évaluation sur l'afficheur (101) après l'affichage de la bonne réponse et de l'écran d'explication sur l'afficheur (101).

# FIG.1

101

102     103     102

# FIG.2

# FIG.3

# FIG.4

```
                    300                   313
              351                     314              202
        FIRST              CAMERA              RIGHT
        CALCULAT-          IF                  CAMERA
        OR
              352                     315              203
        SECOND             CAMERA              LEFT
        CALCULAT-          IF                  CAMERA
        OR
              353                            316
        THIRD
        CALCULAT-
        OR
              354
  101   VISUAL
        LINE               LED DRIVE           103
        DETECTOR           CONTROL-     LED LIGHT
              355          LER          SOURCE
        VISUAL
        POINT
        DETECTOR
              356
        OUTPUT
        CONTROL-
        LER
              357                     322              205
        EVALUATOR          SPEAKER             SPEAKER
                           DRIVER
```

EP 2 979 635 B1

# FIG.5

# FIG.6

# FIG.7

# FIG.8

START

REPRODUCE TARGET IMAGE AT ONE POINT (CENTER) ON DISPLAY ~S101

TURN ON LED ~S102

IMAGE EYE BY LEFT AND RIGHT CAMERAS ~S103

CALCULATE COORDINATES OF PUPIL CENTER AND CORNEAL REFLEX POINT ON IMAGES ~S104

TRANSFORM PUPIL CENTER AND CORNEAL REFLEX POINT FROM COORDINATES OF LEFT AND RIGHT CAMERAS INTO WORLD COORDINATES ~S105

CALCULATE LINE CONNECTING BETWEEN LED CENTRAL POSITION AND CORNEAL REFLEX POINT ~S106

CALCULATE LINE CONNECTING BETWEEN ONE POINT ON DISPLAY REPRODUCING TARGET IMAGE AND PUPIL CENTER ~S107

CALCULATE POINT OF INTERSECTION BETWEEN TWO LINES AS CORNEAL CURVATURE CENTER ~S108

CALCULATE AND STORE DISTANCE BETWEEN PUPIL CENTER AND CORNEAL CURVATURE CENTER ~S109

END

# FIG.9

# FIG.10

```
           ┌─────────────┐
           │    START    │
           └──────┬──────┘
                  ↓
      ┌───────────────────────┐
      │      TURN ON LED       │────S201
      └───────────┬───────────┘
                  ↓
      ┌───────────────────────┐
      │  IMAGE EYE BY LEFT AND │────S202
      │     RIGHT CAMERAS      │
      └───────────┬───────────┘
                  ↓
      ┌───────────────────────┐
      │ CALCULATE COORDINATES OF│
      │    PUPIL CENTER AND     │────S203
      │CORNEAL REFLEX POINT ON IMAGE│
      └───────────┬───────────┘
                  ↓
      ┌───────────────────────┐
      │ TRANSFORM PUPIL CENTER AND│
      │ CORNEAL REFLEX POINT FROM │
      │COORDINATES OF LEFT AND RIGHT│────S204
      │    CAMERAS INTO WORLD     │
      │      COORDINATES         │
      └───────────┬───────────┘
                  ↓
      ┌───────────────────────┐
      │  CALCULATE LINE CONNECTING│
      │ BETWEEN LED CENTRAL POSITION│────S205
      │ AND CORNEAL REFLEX POINT │
      └───────────┬───────────┘
                  ↓
      ┌───────────────────────┐
      │CALCULATE CORNEAL CURVATURE│
      │ CENTER FROM CALCULATED LINE│
      │   AND DISTANCE BETWEEN    │────S206
      │     PUPIL CENTER AND      │
      │  CORNEAL CURVATURE CENTER │
      └───────────┬───────────┘
                  ↓
      ┌───────────────────────┐
      │ CALCULATE VISUAL LINE VECTOR│
      │  CONNECTING BETWEEN PUPIL  │
      │CENTER AND CORNEAL CURVATURE│────S207
      │        CENTER              │
      └───────────┬───────────┘
                  ↓
      ┌───────────────────────┐
      │CALCULATE WORLD COORDINATES│
      │  OF POINT OF INTERSECTION │
      │BETWEEN VISUAL LINE VECTOR AND│────S208
      │        DISPLAY            │
      └───────────┬───────────┘
                  ↓
      ┌───────────────────────┐
      │TRANSFORM WORLD COORDINATES│
      │ INTO COORDINATES OF DISPLAY│────S209
      │   TO ACQUIRE VISUAL POINT │
      └───────────┬───────────┘
                  ↓
           ┌─────────────┐
           │     END     │
           └─────────────┘
```

# FIG.11

EP 2 979 635 B1

# FIG.12

```
                    START

REPRODUCE TARGET IMAGE AT ONE POINT (CENTER) ON     S301
                    DISPLAY

              TURN ON LED                           S302

       IMAGE EYE BY LEFT AND RIGHT CAMERAS          S303

      CALCULATE COORDINATES OF PUPIL CENTER AND     S304
          CORNEAL REFLEX POINT ON IMAGES

 TRANSFORM PUPIL CENTER AND CORNEAL REFLEX POINT    S305
 FROM COORDINATES OF LEFT AND RIGHT CAMERAS INTO
                WORLD COORDINATES

    CALCULATE LINE CONNECTING BETWEEN LED CENTRAL   S306
        POSITION AND CORNEAL REFLEX POINT

   CALCULATE LINE CONNECTING BETWEEN ONE POINT ON   S307
       DISPLAY REPRODUCING TARGET IMAGE AND
                 PUPIL CENTER

 CALCULATE SEGMENT CONNECTING BETWEEN ONE POINT     S308
   ON DISPLAY REPRODUCING TARGET IMAGE AND LED
      CENTRAL POSITION AND LENGTH OF SEGMENT

 CALCULATE SEGMENT PASSING THROUGH PUPIL CENTER     S309
  AND PARALLEL TO SEGMENT DETERMINED AT S308 AND
              LENGTH OF SEGMENT

   CALCULATE DISTANCE BETWEEN CORNEAL CURVATURE     S310
    CENTER AND PUPIL CENTER BASED ON SIMILARITY
 BETWEEN TRIANGLE WITH SEGMENT CALCULATED AT S308
 AS BASE AND TRIANGLE WITH SEGMENT CALCULATED AT
 S309 AS LOWER SIDE WITH CORNEAL CURVATURE CENTER
                  AS VERTEX

          STORE CALCULATED DISTANCE                 S311

                     END
```

# FIG.13

(0, 0)

(Xmax, 0)

H

M

S

(0, Ymax)

(Xmax, Ymax)

C

# FIG.14

(0, 0)

(Xmax, 0)

H

M

S

(0, Ymax)

(Xmax, Ymax)

C

# FIG.15

# FIG.16

# FIG.17

```
            ┌─────────────┐
            │    START    │
            └──────┬──────┘
                   ▼
    ┌──────────────────────────────┐
    │   DISPLAY STILL IMAGE 1 AND   │────S401
    │      DETECT GAZING POINT      │
    └──────────────┬───────────────┘
                   ▼
    ┌──────────────────────────────┐
    │   DISPLAY STILL IMAGE 2 AND   │────S402
    │      DETECT GAZING POINT      │
    └──────────────┬───────────────┘
                   ▼
    ┌──────────────────────────────┐
    │      SELECT PRIMARY ANSWER    │────S403
    └──────────────┬───────────────┘
                   ▼
    ┌──────────────────────────────┐
    │    DISPLAY MOVING IMAGE AND   │────S404
    │      DETECT GAZING POINT      │
    └──────────────┬───────────────┘
                   ▼
    ┌──────────────────────────────┐
    │    SELECT SECONDARY ANSWER    │────S405
    └──────────────┬───────────────┘
                   ▼
    ┌──────────────────────────────┐
    │      DISPLAY RIGHT ANSWER     │────S406
    └──────────────┬───────────────┘
                   ▼
    ┌──────────────────────────────┐
    │      DISPLAY EXPLANATION      │────S407
    └──────────────┬───────────────┘
                   ▼
    ┌──────────────────────────────┐
    │           ANALYZE            │────S408
    └──────────────┬───────────────┘
                   ▼
    ┌──────────────────────────────┐
    │    DISPLAY ANALYSIS RESULT    │────S409
    └──────────────┬───────────────┘
                   ▼
            ┌─────────────┐
            │     END     │
            └─────────────┘
```

# FIG.18

Why does this person lie on the roadside?  —Q

| He felt bad. | —A1 |

| He had an ache in his leg. | —A2 |

| He stumbled over a stone and fell down. | —A3 |

| Somebody told him "Lie down." | —A4 |

# FIG.19

ANSWER                  NEXT ▷ ——2001

He felt bad. ——A1

He had an ache in his leg. ——A2

He stumbled over a stone and fell down. ——A3

Somebody told him "Lie down." ——A4

# FIG.20

EXPLANATION            NEXT ▷ ——2101

A stone was on a road on which a person was walking, and he stumbled over the stone, tumbled, and fell down.

FIG.21

```
                    ( START )
                        │
                        ▼
        ┌──────────────────────────────┐
        │  START REPRODUCTION OF VIDEO  │──S501
        └──────────────────────────────┘
                        │
                        ▼
        ┌──────────────────────────────┐
        │         RESET TIMER           │──S502
        └──────────────────────────────┘
                        │
                        ▼
        ┌──────────────────────────────┐
        │ RESET COUNTERS ST1_M, ST1_H,  │──S503
        │  ST1_C, ST1_S, AND ST1_OT     │
        └──────────────────────────────┘
                        │
                        ▼
        ┌──────────────────────────────┐
        │      DETECT GAZING POINT      │──S504
        └──────────────────────────────┘
                        │
                        ▼
              ╱──── S505 ────╲
        YES  ╱  HAS DETECTION  ╲
     ◄───────  BEEN FAILED?     
              ╲               ╱
               ╲─────────────╱
                    │ NO
                    ▼
        ┌──────────────────────────────┐
        │    ACQUIRE COORDINATES OF     │──S506
        │        GAZING POINT           │
        └──────────────────────────────┘
                    │
                    ▼
              ╱─ S507 ─╲        YES
             ╱  AREA M?  ╲──────────────┐
              ╲         ╱                │
               ╲───────╱                 ▼
                 │ NO               ╱─ S508 ─╲    YES
                 │                 ╱  AREA H?  ╲──────────┐
                 │                  ╲         ╱            │
                 │                   ╲───────╱             │
                 │                     │ NO  ─S509          │
                 │                     ▼                    │
                 │         ┌──────────────────────────┐     │
                 │         │  COUNT UP COUNTER ST1_M   │     │
                 │         └──────────────────────────┘     │
                 │                                          ▼
                 │                          ┌──────────────────────────┐
                 │                          │ COUNT UP COUNTER ST1_H    │──S510
                 │                          └──────────────────────────┘
                 ▼
              ╱─ S511 ─╲        YES
             ╱  AREA C?  ╲──────────────┐
              ╲         ╱                ▼
               ╲───────╱          ┌──────────────────────────┐
                 │ NO             │ COUNT UP COUNTER ST1_C    │──S512
                 │                └──────────────────────────┘
                 ▼
              ╱─ S513 ─╲        YES
             ╱  AREA S?  ╲──────────────┐
              ╲         ╱                ▼
               ╲───────╱          ┌──────────────────────────┐
                 │ NO             │ COUNT UP COUNTER ST1_S    │──S514
                 │                └──────────────────────────┘
                 ▼
        ┌──────────────────────────────┐
        │   COUNT UP COUNTER ST1_OT     │──S515
        └──────────────────────────────┘
                        │
                        ▼
              ╱──── S516 ────╲
        NO   ╱      HAS        ╲
     ◄───────  TIMER REACHED   
              ╲   TIME-OUT?    ╱
               ╲─────────────╱
                    │ YES
                    ▼
        ┌──────────────────────────────┐
        │  STOP REPRODUCTION OF VIDEO   │──S517
        └──────────────────────────────┘
                        │
                        ▼
                    (  END  )
```

39

# FIG.22

```
                    START

                      │
                      ▼
              ┌─────────────────┐  ⌐S601
              │   IS ANSWER     │          YES
              │ RIGHT ANSWER?   │─────────────────────┐
              └─────────────────┘                     │
                      │                               ▼      ⌐S602
                      │ NO              ┌────────────────────────────────┐
                      │                 │ CAPACITY OF UNDERSTANDING      │
                      │                 │ CAUSAL RELATIONS IS HIGH       │
                      │◀────────────────└────────────────────────────────┘
                      ▼
              ┌─────────────────┐
              │ ans1=ST1_M+ST2_M│  ～S603
              └─────────────────┘
                      │
                      ▼
              ┌─────────────────┐  ⌐S604
              │    ans1>k11?    │          YES
              └─────────────────┘─────────────────────┐
                      │                               ▼      ⌐S605
                      │ NO              ┌────────────────────────────────┐
                      │                 │ ATTENTION TO CHANGES IN        │
                      │                 │ EVENTS IS HIGH                 │
                      │◀────────────────└────────────────────────────────┘
                      ▼
              ┌─────────────────┐
              │ ans2=ST1_H+ST2_H│  ～S606
              └─────────────────┘
                      │
                      ▼
              ┌─────────────────┐  ⌐S607
              │    ans2>k12?    │          YES
              └─────────────────┘─────────────────────┐
                      │                               ▼      ⌐S608
                      │ NO              ┌────────────────────────────────┐
                      │                 │      SOCIALITY IS HIGH         │
                      │◀────────────────└────────────────────────────────┘
                      ▼
              ┌─────────────────┐
              │ ans3=ST1_C+ST2_C│  ～S609
              └─────────────────┘
                      │
                      ▼
              ┌─────────────────┐  ⌐S610
              │    ans3>k13?    │          YES
              └─────────────────┘─────────────────────┐
                      │                               ▼      ⌐S611
                      │ NO              ┌────────────────────────────────┐
                      │                 │ CAPACITY OF PREDICTING         │
                      │                 │ RELEVANCE IS HIGH              │
                      │◀────────────────└────────────────────────────────┘
                      ▼
       ┌─────────────────────────────┐
       │ ans4=ST1_M+ST2_M+ST1_C+     │  ～S612
       │ ST2_C+ST1_S+ST2_S           │
       └─────────────────────────────┘
                      │
                      ▼
              ┌─────────────────┐  ⌐S613
              │    ans4>k14?    │          YES
              └─────────────────┘─────────────────────┐
                      │                               ▼      ⌐S614
                      │ NO              ┌────────────────────────────────┐
                      │                 │  DEGREE OF INTEREST IS HIGH    │
                      │◀────────────────└────────────────────────────────┘
                      ▼
                     END
```

# FIG.23

| CAUSE | RESULT |
|---|---|
| A banana peel | Slipping and tumbling |
| A stone | Stumbling and tumbling |
| Pepper | A sneeze |
| Heavy baggage | A lower-back pain |
| Throwing a ball | Glass breaks |
| Being late | Missing the train |
| Stepping a tail of a dog | Getting barked at |
| Slipping from one's hand | A vase falls and breaks |
| Not carrying an umbrella | Getting wet |
| A car passes by | Splashing and getting wet |
| Getting stung by a bee | Getting swollen |
| Seeing something fearful | A baby cries |
| Eating a confection | Getting delighted |
| A present | Getting delighted |
| Parting with a friend | Crying |
| Eating sweets | Crying with decayed teeth |
| Taking off one's clothes | Feeling cold |
| Applying colors | A kennel has been completed |
| Hitting with a hammer | A nail is knocked |
| Getting hit by a ball | Crying |
| A car hits | Breaking |
| A rope of a swing breaks | Falling |
| Slipping one's hand off a thread | A balloon flies away |
| A guest comes | Getting a present |
| Drinking juice | A cup becomes empty |
| Getting barked at by a dog | Crying |
| Having a meal | Being full to bursting |
| Eating too much | Having a stomachache |
| Orange picking | Oranges run out |
| Tumbling | Grazing one's shin |

# FIG.24

START

STORE SUBJECT INFORMATION AND
STORE MEASUREMENT DATE — S701

MEASURE — S702

S703
ARE THERE ANY
MEASUREMENT DATA OF
SAME SUBJECT?            YES

NO

S704
DISPLAY PAST MEASUREMENT DATA
AND DISPLAY CHANGES

DISPLAY PRESENT MEASUREMENT
DATA — S705

END

# FIG.25

| | PREVIOUS | THIS TIME | CHANGE |
|---|---|---|---|
| STILL IMAGE | CAPACITY OF UNDERSTANDING CAUSAL RELATIONS IS HIGH | CAPACITY OF UNDERSTANDING CAUSAL RELATIONS IS HIGH | |
| | GAZING AT CHANGES ans1_old | GAZING AT CHANGES ans1_new | GAZING AT CHANGES ans1_new-ans1_old |
| | SOCIALITY IS HIGH ans2_old | SOCIALITY IS HIGH ans2_new | SOCIALITY IS HIGH ans2_new-ans2_old |
| | CAPACITY OF PREDICTING RELEVANCE IS HIGH ans3_old | CAPACITY OF PREDICTING RELEVANCE IS HIGH ans3_new | CAPACITY OF PREDICTING RELEVANCE IS HIGH ans3_new-ans3_old |
| | DEGREE OF INTEREST IS HIGH ans4_old | DEGREE OF INTEREST IS HIGH ans4_new | DEGREE OF INTEREST IS HIGH ans4_new-ans4_old |
| MOVING IMAGE | CAPACITY OF UNDERSTANDING CAUSAL RELATIONS IS HIGH | CAPACITY OF UNDERSTANDING CAUSAL RELATIONS IS HIGH | |
| | GAZING AT CHANGES ans1_old | GAZING AT CHANGES ans1_new | GAZING AT CHANGES ans1_new-ans1_old |
| | SOCIALITY IS HIGH ans2_old | SOCIALITY IS HIGH ans2_new | SOCIALITY IS HIGH ans2_new-ans2_old |
| | CAPACITY OF PREDICTING RELEVANCE IS HIGH ans3_old | CAPACITY OF PREDICTING RELEVANCE IS HIGH ans3_new | CAPACITY OF PREDICTING RELEVANCE IS HIGH ans3_new-ans3_old |
| | DEGREE OF INTEREST IS HIGH ans4_old | DEGREE OF INTEREST IS HIGH ans4_new | DEGREE OF INTEREST IS HIGH ans4_new-ans4_old |

EP 2 979 635 B1

# FIG.26

Flowchart:

START

→ S801 IS ANSWER RIGHT ANSWER?
- YES → S802 CAPACITY OF UNDERSTANDING CAUSAL RELATIONS IS HIGH
- NO

ans1=MOV_M — S803

→ S804 ans1>k21?
- YES → S805 ATTENTION TO CHANGES IN EVENTS IS HIGH
- NO

ans2=MOV_H — S806

→ S807 ans2>k22?
- YES → S808 SOCIALITY IS HIGH
- NO

ans3=MOV_C — S809

→ S810 ans3>k23?
- YES → S811 CAPACITY OF PREDICTING RELEVANCE IS HIGH
- NO

ans4=MOV_M+MOV_C+MOV_S — S812

→ S813 ans4>k24?
- YES → S814 DEGREE OF INTEREST IS HIGH
- NO

END

# FIG.27

START

S901

DISPLAY MENU

S902

HAS END
BUTTON BEEN
PRESSED?  —— YES

NO

S903

HAS SELECTION
NO —— BUTTON BEEN
PRESSED?

YES ~S904

RECEIVE SELECTION OF
QUESTION

S905

DISPLAY STILL IMAGE 1

S906

DISPLAY STILL IMAGE 2

S907

RECEIVE SELECTION OF
ANSWER

S908

DISPLAY RIGHT ANSWER

S909

DISPLAY EXPLANATION

S910

DISPLAY MOVING IMAGE

END

# FIG.28

Select a question.

| QUESTION 1 (2801) | QUESTION 2 (2803) | QUESTION 3 (2803) |
|---|---|---|
| QUESTION 4 (2804) | QUESTION 5 (2805) | QUESTION 6 (2806) |

2802

END ~2811

# FIG.29

This

# FIG.30

turned out like this.
Why?

# FIG.31

Why does this person lie on the roadside? — Q

| | |
|---|---|
| He felt bad. | ～A1 |
| He had an ache in his leg. | ～A2 |
| He stumbled over a stone and fell down. | ～A3 |
| Somebody told him "Lie down." | ～A4 |

# FIG.32

Well done. You're right. ○

NEXT ▷ —2001

ANSWER

| He felt bad. | —A1 |

| He had an ache in his leg. | —A2 |

| He stumbled over a stone and fell down. | —A3 |

| Somebody told him "Lie down." | —A4 |

# FIG.33

EXPLANATION

A stone was on a road on which a person was walking, and he stumbled over the stone, tumbled, and fell down.

REPRODUCE
ANSWER VIDEO —3301

# FIG.34

He stumbled over a stone.

# FIG.35

This

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011206542 A
- US 20100004977 A
- EP 2754397 A

**Non-patent literature cited in the description**

- **PIERCE K et al.** Preference for Geometric Patterns Early in Life as a Risk Factor for Autism. *Arch Gen Psychiatry,* January 2011, vol. 68 (1), 101-109